# EUROPEAN PATENT APPLICATION

(11) **EP 2 722 342 A1**
(43) Date of publication of application: **23.04.2014**
(21) Application number: 12189212.9
(22) Date of filing: 19.10.2012
(51) Int. Cl.: C07K 16/28, C07K 16/30, A61K 39/395, A61P 35/00, G01N 33/50

(54) **Methods and compositions for the treatment of pancreatic cancer**

(71) Applicant: Opsona Therapeutics Limited, Dublin 2 (IE)
(72) Inventor: The designation of the inventor has not yet been filed
(74) Representative: Carr, Anne Marie

(57) **Abstract**

A composition comprising a TLR2 antagonist for use in the treatment or prophylaxis of pancreatic cancer is provided. In particular, the composition comprises a TLR2 antagonistic antibody which binds specifically to a non-continuous epitope comprising amino acid residues His318, Pro320, Arg321, Tyr323, Lys347, Phe349, Leu371, Glu375, Tyr376 and His398 of human TLR2. Also provided is a pharmaceutical composition comprising the TLR2 antagonist and the chemotherapeutic agent gemcitabine, and further provided is a screening method for the identification of compounds for use in treatment or prevention of pancreatic cancer.

SEQUENCE LISTING

<110>  Opsona Therapeutics Limited

<120>  Methods and Compositions for the Treatment of Pancreatic Cancer

<130>  P126841.EP.01

<160>  13

<170>  PatentIn version 3.5

<210>  1
<211>  784
<212>  PRT
<213>  Homo sapiens

<400>  1

<210>  2
<211>  784
<212>  PRT
<213>  Mus musculus

<400>  2

Met Leu Arg Ala Leu Trp Leu Phe Trp Ile Leu Val Ala Ile Thr Val
1               5                   10                  15

Leu Phe Ser Lys Arg Cys Ser Ala Gln Glu Ser Leu Ser Cys Asp Ala
            20                  25                  30

Ser Gly Val Cys Asp Gly Arg Ser Arg Ser Phe Thr Ser Ile Pro Ser
        35                  40                  45

Gly Leu Thr Ala Ala Met Lys Ser Leu Asp Leu Ser Phe Asn Lys Ile
    50                  55                  60

Thr Tyr Ile Gly His Gly Asp Leu Arg Ala Cys Ala Asn Leu Gln Val
65                  70                  75                  80

Leu Met Leu Lys Ser Ser Arg Ile Asn Thr Ile Glu Gly Asp Ala Phe
                85                  90                  95

Tyr Ser Leu Gly Ser Leu Glu His Leu Asp Leu Ser Asp Asn His Leu
            100                 105                 110

Ser Ser Leu Ser Ser Ser Trp Phe Gly Pro Leu Ser Ser Leu Lys Tyr
        115                 120                 125

Leu Asn Leu Met Gly Asn Pro Tyr Gln Thr Leu Gly Val Thr Ser Leu
    130                 135                 140

Phe Pro Asn Leu Thr Asn Leu Gln Thr Leu Arg Ile Gly Asn Val Glu
145                 150                 155                 160

Thr Phe Ser Glu Ile Arg Arg Ile Asp Phe Ala Gly Leu Thr Ser Leu
                165                 170                 175

Asn Glu Leu Glu Ile Lys Ala Leu Ser Leu Arg Asn Tyr Gln Ser Gln
            180                 185                 190

Ser Leu Lys Ser Ile Arg Asp Ile His His Leu Thr Leu His Leu Ser
        195                 200                 205

Glu Ser Ala Phe Leu Leu Glu Ile Phe Ala Asp Ile Leu Ser Ser Val
    210                 215                 220

Arg Tyr Leu Glu Leu Arg Asp Thr Asn Leu Ala Arg Phe Gln Phe Ser
225                 230                 235                 240

Pro Leu Pro Val Asp Glu Val Ser Ser Pro Met Lys Lys Leu Ala Phe
                245                 250                 255

Arg Gly Ser Val Leu Thr Asp Glu Ser Phe Asn Glu Leu Leu Lys Leu
            260                 265                 270

Leu Arg Tyr Ile Leu Glu Leu Ser Glu Val Glu Phe Asp Asp Cys Thr
        275                 280                 285

Leu Asn Gly Leu Gly Asp Phe Asn Pro Ser Glu Ser Asp Val Val Ser
    290                 295                 300

Glu Leu Gly Lys Val Glu Thr Val Thr Ile Arg Arg Leu His Ile Pro
305                 310                 315                 320

Gln Phe Tyr Leu Phe Tyr Asp Leu Ser Thr Val Tyr Ser Leu Leu Glu
                325                 330                 335

Lys Val Lys Arg Ile Thr Val Glu Asn Ser Lys Val Phe Leu Val Pro
            340                 345                 350

Cys Ser Phe Ser Gln His Leu Lys Ser Leu Glu Phe Leu Asp Leu Ser
        355                 360                 365

Glu Asn Leu Met Val Glu Glu Tyr Leu Lys Asn Ser Ala Cys Lys Gly
    370                 375                 380

Ala Trp Pro Ser Leu Gln Thr Leu Val Leu Ser Gln Asn His Leu Arg
385                 390                 395                 400

Ser Met Gln Lys Thr Gly Glu Ile Leu Leu Thr Leu Lys Asn Leu Thr
                405                 410                 415

Ser Leu Asp Ile Ser Arg Asn Thr Phe His Pro Met Pro Asp Ser Cys
            420                 425                 430

Gln Trp Pro Glu Lys Met Arg Phe Leu Asn Leu Ser Ser Thr Gly Ile
        435                 440                 445

Arg Val Val Lys Thr Cys Ile Pro Gln Thr Leu Glu Val Leu Asp Val
    450                 455                 460

Ser Asn Asn Asn Leu Asp Ser Phe Ser Leu Phe Leu Pro Arg Leu Gln
465                 470                 475                 480

Glu Leu Tyr Ile Ser Arg Asn Lys Leu Lys Thr Leu Pro Asp Ala Ser
                485                 490                 495

Leu Phe Pro Val Leu Leu Val Met Lys Ile Arg Glu Asn Ala Val Ser
            500                 505                 510

Thr Phe Ser Lys Asp Gln Leu Gly Ser Phe Pro Lys Leu Glu Thr Leu
        515                 520                 525

Glu Ala Gly Asp Asn His Phe Val Cys Ser Cys Glu Leu Leu Ser Phe
    530                 535                 540

Thr Met Glu Thr Pro Ala Leu Ala Gln Ile Leu Val Asp Trp Pro Asp
545                 550                 555                 560

Ser Tyr Leu Cys Asp Ser Pro Pro Arg Leu His Gly His Arg Leu Gln
                565                 570                 575

Asp Ala Arg Pro Ser Val Leu Glu Cys His Gln Ala Ala Leu Val Ser
            580                 585                 590

Gly Val Cys Cys Ala Leu Leu Leu Leu Ile Leu Leu Val Gly Ala Leu
        595                 600                 605

Cys His His Phe His Gly Leu Trp Tyr Leu Arg Met Met Trp Ala Trp
    610                 615                 620

Leu Gln Ala Lys Arg Lys Pro Lys Lys Ala Pro Cys Arg Asp Val Cys
625                 630                 635                 640

Tyr Asp Ala Phe Val Ser Tyr Ser Glu Gln Asp Ser His Trp Val Glu
                645                 650                 655

Asn Leu Met Val Gln Gln Leu Glu Asn Ser Asp Pro Pro Phe Lys Leu
            660                 665                 670

Cys Leu His Lys Arg Asp Phe Val Pro Gly Lys Trp Ile Ile Asp Asn
        675                 680                 685

Ile Ile Asp Ser Ile Glu Lys Ser His Lys Thr Val Phe Val Leu Ser
    690                 695                 700

Glu Asn Phe Val Arg Ser Glu Trp Cys Lys Tyr Glu Leu Asp Phe Ser
705                 710                 715                 720

His Phe Arg Leu Phe Asp Glu Asn Asn Asp Ala Ala Ile Leu Val Leu
                725                 730                 735

Leu Glu Pro Ile Glu Arg Lys Ala Ile Pro Gln Arg Phe Cys Lys Leu
            740                 745                 750

Arg Lys Ile Met Asn Thr Lys Thr Tyr Leu Glu Trp Pro Leu Asp Glu
        755                 760                 765

Gly Gln Gln Glu Val Phe Trp Val Asn Leu Arg Thr Ala Ile Lys Ser
    770                 775                 780

<210>  3
<211>  8
<212>  PRT
<213>  Mus musculus

<400>  3

Gly Phe Thr Phe Thr Thr Tyr Gly
1               5

<210>  4
<211>  8
<212>  PRT
<213>  Mus musculus

<400>  4

Ile Tyr Pro Arg Asp Gly Ser Thr
1               5

<210>  5
<211>  11
<212>  PRT
<213>  Mus musculus

<400>  5

Ala Arg Leu Thr Gly Gly Thr Phe Leu Asp Tyr
1               5                   10

<210>  6
<211>  10
<212>  PRT
<213>  Mus musculus

<400>  6

Glu Ser Val Glu Tyr Tyr Gly Thr Ser Leu
1               5                   10


<210>  7
<211>  9
<212>  PRT
<213>  Mus musculus

<400>  7

Gln Gln Ser Arg Lys Leu Pro Trp Thr
1               5


<210>  8
<211>  167
<212>  PRT
<213>  Mus musculus

<400>  8

Met Ser Ser Pro Gln Ser Leu Lys Thr Leu Ile Leu Thr Met Gly Trp
1               5                   10                  15

Ser Trp Ile Phe Leu Phe Leu Leu Ser Gly Thr Ala Gly Val His Ser
            20                  25                  30

Gln Val Gln Leu Gln Gln Ser Gly Pro Glu Leu Val Asn Pro Gly Ala
        35                  40                  45

Ser Val Lys Leu Ser Cys Lys Ala Ser Gly Phe Thr Phe Thr Thr Tyr
    50                  55                  60

Gly Ile Asn Trp Val Lys Gln Gly Pro Gly Gln Gly Leu Glu Trp Ile
65                  70                  75                  80

Gly Trp Ile Tyr Pro Arg Asp Gly Ser Thr Asn Phe Asn Glu Asn Phe
                85                  90                  95

Lys Asp Lys Ala Ala Leu Thr Val Asp Thr Ser Ser Ser Thr Ala Tyr
            100                 105                 110

Met Glu Leu His Ser Leu Thr Ser Glu Asp Ser Ala Val Tyr Phe Cys
        115                 120                 125

Ala Arg Leu Thr Gly Gly Thr Phe Leu Asp Tyr Trp Gly Gln Gly Thr
    130                 135                 140

Thr Leu Thr Val Ser Ser Ala Lys Thr Thr Pro Pro Ser Val Tyr Pro
145                 150                 155                 160

Leu Ala Pro Gly Ser Ala Ala
                165

<210>  9
<211>  147
<212>  PRT
<213>  Mus musculus

<400>  9

Met Glu Ser Asp Thr Leu Leu Leu Trp Val Leu Leu Leu Trp Val Pro
1               5                   10                  15

Gly Ser Thr Gly Asp Ile Val Leu Thr Gln Ser Pro Ala Ser Leu Ala
            20                  25                  30

Val Ser Leu Gly Gln Arg Ala Thr Ile Ser Cys Arg Ala Ser Glu Ser
        35                  40                  45

Val Glu Tyr Tyr Gly Thr Ser Leu Met Gln Trp Tyr Gln Gln Lys Pro
    50                  55                  60

Gly Gln Pro Pro Lys Leu Leu Ile Phe Gly Ala Ser Asn Val Glu Ser
65                  70                  75                  80

Gly Val Pro Val Arg Phe Ser Gly Ser Gly Ser Gly Thr Asp Phe Ser
                85                  90                  95

Leu Asn Ile His Pro Val Glu Glu Asp Asp Ile Val Met Tyr Phe Cys
            100                 105                 110

Gln Gln Ser Arg Lys Leu Pro Trp Thr Phe Gly Gly Gly Thr Lys Leu
        115                 120                 125

Glu Ile Lys Arg Ala Asp Ala Ala Pro Thr Val Ser Ile Phe Pro Pro
    130                 135                 140

Ser Ser Glu
145

<210>  10
<211>  111
<212>  PRT
<213>  Homo sapiens

<400>  10

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Glu Tyr Tyr
            20                  25                  30

Gly Thr Ser Leu Met Gln Trp Tyr Gln Gln Lys Pro Gly Gln Pro Pro
        35                  40                  45

Lys Leu Leu Ile Phe Gly Ala Ser Asn Val Glu Ser Gly Val Pro Asp
    50                  55                  60

Arg Phe Ser Gly Ser Gly Ser Gly Thr Asp Phe Thr Leu Lys Ile Ser
65                  70                  75                  80

Arg Val Glu Ala Glu Asp Val Gly Met Tyr Phe Cys Gln Gln Ser Arg
                85                  90                  95

Lys Leu Pro Trp Thr Phe Gly Gly Gly Thr Lys Val Glu Ile Lys
            100                 105                 110

<210> 11
<211> 118
<212> PRT
<213> Homo sapiens

<400> 11

Gln Val Gln Leu Val Gln Ser Gly Ser Glu Leu Lys Lys Pro Gly Ala
1               5                   10                  15

Ser Val Lys Leu Ser Cys Lys Ala Ser Gly Phe Thr Phe Thr Thr Tyr
            20                  25                  30

Gly Ile Asn Trp Val Arg Gln Ala Pro Gly Gln Gly Leu Glu Trp Ile
        35                  40                  45

Gly Trp Ile Tyr Pro Arg Asp Gly Ser Thr Asn Phe Asn Glu Asn Phe
    50                  55                  60

Lys Asp Arg Ala Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Phe Cys
                85                  90                  95

Ala Arg Leu Thr Gly Gly Thr Phe Leu Asp Tyr Trp Gly Gln Gly Thr
            100                 105                 110

Thr Val Thr Val Ser Ser
        115

<210> 12
<211> 218
<212> PRT
<213> Homo sapiens

<400>  12

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Glu Tyr Tyr
            20                  25                  30

Gly Thr Ser Leu Met Gln Trp Tyr Gln Gln Lys Pro Gly Gln Pro Pro
        35                  40                  45

Lys Leu Leu Ile Phe Gly Ala Ser Asn Val Glu Ser Gly Val Pro Asp
    50                  55                  60

Arg Phe Ser Gly Ser Gly Ser Gly Thr Asp Phe Thr Leu Lys Ile Ser
65                  70                  75                  80

Arg Val Glu Ala Glu Asp Val Gly Met Tyr Phe Cys Gln Gln Ser Arg
                85                  90                  95

Lys Leu Pro Trp Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  13
<211>  445
<212>  PRT
<213>  Homo sapiens

<400>  13

Gln Val Gln Leu Val Gln Ser Gly Ser Glu Leu Lys Lys Pro Gly Ala
1               5                   10                  15

Ser Val Lys Leu Ser Cys Lys Ala Ser Gly Phe Thr Phe Thr Thr Tyr
            20                  25                  30

Gly Ile Asn Trp Val Arg Gln Ala Pro Gly Gln Gly Leu Glu Trp Ile
        35                  40                  45

Gly Trp Ile Tyr Pro Arg Asp Gly Ser Thr Asn Phe Asn Glu Asn Phe
    50                  55                  60

Lys Asp Arg Ala Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Phe Cys
                85                  90                  95

Ala Arg Leu Thr Gly Gly Thr Phe Leu Asp Tyr Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln

260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Arg Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys
        435                 440                 445

## Description

### Field of the Invention

The present invention relates to a method for the treatment or prevention of pancreatic cancer. Also provided are compositions for use in the treatment or prevention of pancreatic cancer.

### Background to the Invention

Toll-like Receptors (TLRs) form a family of pattern recognition receptors which have a key role in activating the innate immune response. Eleven TLRs have been identified in humans to date. The members of the TLR family are highly conserved, with most mammalian species having between ten to fifteen TLRs. Each TLR recognises specific pathogen-associated molecular signatures. Toll-like Receptor 2 (TLR2, CD282, TLR-2) is activated by peptidoglycan, lipoproteins and lipoteichoic acid. TLR2 is known to dimerise into two functional heterodimers. In particular, TLR2 is known to form a heterodimer with either Toll-like Receptor 1 (TLR1, TLR-1) or Toll-like Receptor 6 (TLR6, TLR-6). It is possible that further heterodimers are formed with Toll-like Receptor 4 (TLR4, TLR-4) and Toll-like Receptor 10 (TLR10, TLR-10). In addition to microbial derived components, TLRs are also known to recognize damage-associated molecular patterns (DAMPs). These are host endogenous molecules released and distributed following stress, tissue damage and cellular disease. WO 2005/028509 discloses a murine IgG1 anti-TLR2 antibody which was derived from hybridoma clone T2.5 (HyCult Biotechnology b.v., Cell Sciences, Canton, USA: catalogue number 1054 - also known as OPN-301). WO2011/003925 describes a humanized version of T2.5 which is designated OPN-305.

Pancreatic cancer is the fourth most common cause of cancer related deaths in the United States and the eighth most common worldwide. It has one of the highest fatality rates of all cancers and is the fourth highest cancer killer among men and women. For all stages combined, the 1- and 5-year relative survival rates are 25% and 6% respectively. For local disease, the 5-year survival rate is approximately 20%. The median survival rates for locally advanced and for metastatic disease, which collectively represent over 80% of individuals, are about 10 and 6 months respectively.

Treatment of pancreatic cancer depends on the stage of the cancer. Although only localized cancer is considered suitable for surgery with curative intent at present, only ~20% of cases present with localised disease at diagnosis. Surgery can also be performed for palliation if the malignancy is invading or compressing the duodenum or colon. In such cases, bypass surgery might overcome the obstruction and improve quality of life, but is not intended as a cure. In patients not suitable for resection with curative intent, palliative chemotherapy may be used to improve quality of life and gain a modest survival benefit. Gemcitabine was approved by the United States Food and Drug Administration in 1998 after a clinical trial reported improvements in quality of life and a 5-week improvement in median survival duration in patients with advanced pancreatic cancer. Gemcitabine has multiple immunostimulatory effects. It enhances antigen presentation by inducing tumour apoptosis and eliminates myeloid-derived suppressor cells. Cyclophosphamide enhances anti-tumour immunity by reducing the immunosuppressive effects of CD4+CD25+ regulatory T cells.

There is a need for improved methods for treating pancreatic cancer, in particular, metastatic pancreatic cancer. Metastasis is the leading cause of mortality in cancer patients. However, there are no effective therapies to target the development and progression of metastases in pancreatic cancer.

### Summary of the Invention

According to a first aspect of the present invention there is provided a method of treating or preventing pancreatic cancer comprising the step of:
- administering a therapeutically effective amount of a Toll-like receptor 2 (TLR2) antagonist to a subject in need thereof.

Typically the treatment of the present invention inhibits or reduces tumour progression and increases apoptosis leading to enhanced survival of, and/or an improved quality of life for, the subject. The treatment may further enhance an immunostimulatory response and/or reduce immunosuppressive effects, for example, the immunosuppressive effects of regulatory T cells. Without wishing to be bound by theory, the effect observed may also involve targeting a target present on tumour cells as the present inventors have shown that TLR2 is also expressed by malignant cells.

Typically the TLR2 antagonist has binding specificity to TLR2, typically human TLR2. Typically the TLR2 antagonist has binding specificity to an epitope comprising amino acid residues of the extracellular domain of TLR2. The extracellular domain of the human form of TLR2 comprises 587 amino acid resides, specifically amino acids 1-587 of the 784 amino acid full length human TLR2 sequence shown as SEQ ID NO:1 and defined as Genbank Accession Number AAC 34133 (URL www.ncbi.nlm.nih.gov). Typically the binding member binds to the ligand binding region of TLR2. Typically the binding member inhibits or prevents dimerization of TLR2 with Toll-like Receptor 1 (TLR1) or Toll-like Receptor 6 (TLR6) by covering the interaction surface between TLR2 and TLR1 or TLR6. Typically the binding region/interaction surface is located within leucine-rich repeat (LRR) regions 11 to 14 of TLR2. In certain embodiments the TLR2 antagonist has binding specificity to an epitope comprising, consisting of or consisting essentially of LRR regions 11 to 14 of TLR2, or a sequence which has at least 85%, 90% or 95% sequence identity thereto.

In certain embodiments the TLR2 antagonist binds to a non-continuous epitope comprising, consisting of or consisting essentially of amino acid residues His318 (H, histidine), Pro320 (P, proline), Arg321 (R, arginine) or Gln321 (Q, glutamine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of SEQ ID NO: 1 or SEQ ID NO:2.

In certain embodiments the TLR2 antagonist binds to a non-continuous epitope comprising, consisting of or consisting essentially of amino acid residues His318 (H, histidine), Pro320 (P, proline), Arg321 (R, arginine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of SEQ ID NO: 1.

### SEQ ID NO:1 (human TLR2)

In certain embodiments the TLR2 antagonist binds to a non-continuous epitope comprising, consisting of or consisting essentially of amino acid residues His318 (H, histidine), Pro320 (P, proline), Gln321 (Q, glutamine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of murine TLR2 (SEQ ID NO: 2). SEQ ID NO:2 shows the amino acid murine TLR2 sequence defined as Genbank Accession Number NP_036035 (Mus musculus).

### SEQ ID NO:2 (murine TLR2)

MLRALWLFWI LVAITVLFSK RCSAQESLSC DASGVCDGRS RSFTSIPSGL TAAMKSLDLS
FNKITYIGHG DLRACANLQV LMLKSSRINT IEGDAFYSLG SLEHLDLSDN HLSSLSSSWF
GPLSSLKYLN LMGNPYQTLG VTSLFPNLTN LQTLRIGNVE TFSEIRRIDF AGLTSLNELE
IKALSLRNYQ SQSLKSIRDI HHLTLHLSES AFLLEIFADI LSSVRYLELR DTNLARFQFS
PLPVDEVSSP MKKLAFRGSV LTDESFNELL KLLRYILELS EVEFDDCTLN GLGDFNPSES
DVVSELGKVE TVTIRRLHIP QFYLFYDLST VYSLLEKVKR ITVENSKVFL VPCSFSQHLK
SLEFLDLSEN LMVEEYLKNS ACKGAWPSLQ TLVLSQNHLR SMQKTGEILL
TLKNLTSLDI SRNTFHPMPD SCQWPEKMRF LNLSSTGIRV VKTCIPQTLE
VLDVSNNNLD SFSLFLPRLQ ELYISRNKLK TLPDASLFPV LLVMKIRENA VSTFSKDQLG
SFPKLETLEA GDNHFVCSCE LLSFTMETPA LAQILVDWPD SYLCDSPPRL
HGHRLQDARP SVLECHQAAL VSGVCCALLL LILLVGALCH HFHGLWYLRM
MWAWLQAKRK PKKAPCRDVC YDAFVSYSEQ DSHWVENLMV QQLENSDPPF
KLCLHKRDFV PGKWIIDNII DSIEKSHKTV FVLSENFVRS EWCKYELDFS
HFRLFDENND AAILVLLEPI ERKAIPQRFC KLRKIMNTKT YLEWPLDEGQ
QEVFWVNLRT AIKS

The above identified epitope in humans or mice is a functional epitope for receptor dimerization or inhibition thereof. Typically the epitope is bound by the TLR2 antagonistic antibodies T2.5 and OPN-305. Binding of the identified epitope by a binding member, such as an antibody, results in antagonism of TLR2 biological function, in particular activation and signalling. In particular, binding by the binding member serves to inhibit activation of the TLR2 receptor, irrespective of whether a TLR2 heterodimer is formed with another TLR, such as TLR1, TLR6, TLR4 or TLR10. Furthermore, antibodies binding to this epitope have been shown to cross-react with TLR2 from human, pig and monkey, indicating this to be a critical epitope in a highly conserved region of TLR2.

In certain embodiments the TLR2 antagonist is selected from the group consisting of a protein, a peptide, an antibody or an antigen binding fragment thereof, a peptidomimetic, a nucleic acid, a carbohydrate, a lipid and a small molecule.

In certain embodiments the TLR2 antagonist is an antibody or an antigen binding fragment thereof. Typically the antibody has binding specificity to human TLR2. Typically the antibody has binding specificity to an epitope comprising amino acid residues of the extracellular domain of TLR2. In certain embodiments the antibody binds to the epitope identified above on human TLR2 and/or murine TLR2.

In certain embodiments the antibody is selected from the group consisting of a human antibody, a humanised antibody, a chimeric antibody, a monoclonal antibody, a polyclonal antibody, a synthetic antibody, a camelid antibody, a shark antibody and an *in-vitro* antibody. In certain embodiments an antigen binding fragment may be used. The antigen binding fragment may be derived from any of the aforementioned antibodies. In certain embodiments the antigen binding fragment is selected from the group consisting of a Fab fragment, a scFv fragment, a Fv fragment and a dAb fragment. In certain embodiments the antibody comprises two complete heavy chains and two complete light chains, or an antigen binding fragment thereof. In certain embodiments the antibody is of the isotype IgG, IgA, IgE or IgM. In certain embodiments where the antibody is of the isotype IgG, the antibody may be of the subtype IgG1, IgG2 or IgG3. The antibody may bind to an inhibitory epitope present on TLR2 with a dissociation constant (Kd) of from about 10⁻⁷M to about 10⁻¹¹M. In certain embodiments the antibody is an isolated antibody.

In certain embodiments the antibody or antigen binding fragment comprises a heavy chain variable region comprising a complementarity determining region 1 (CDR1) region comprising the amino acid sequence Gly-Phe-Thr-Phe-Thr-Thr-Tyr-Gly (SEQ ID NO:3), a CDR2 region comprising the amino acid sequence Ile-Tyr-Pro-Arg-Asp-Gly-Ser-Thr (SEQ ID NO:4) and a CDR3 region comprising the amino acid sequence Ala-Arg-Leu-Thr-Gly-Gly-Thr-Phe-Leu-Asp-Tyr (SEQ ID NO:5), and/or a light chain variable region comprising a CDR1 region comprising the amino acid sequence Glu-Ser-Val-Glu-Tyr-Tyr-Gly-Thr-Ser-Leu (SEQ ID NO:6), a CDR2 region comprising the amino acid sequence Gly-Ala-Ser and a CDR3 region comprising the amino acid sequence Gln-Gln-Ser-Arg-Lys-Leu-Pro-Trp-Thr (SEQ ID NO:7).

In certain embodiments the heavy chain variable region comprises the amino acid sequence as depicted in SEQ ID NO:8, or a sequence which has at least 85%, 90%, 95% or 99% amino acid sequence identity over a length of at least 20 amino acids of the amino acid sequence of SEQ ID NO:8, and/or the light chain variable region comprises the amino acid sequence as depicted in SEQ ID NO:9, or a sequence which has at least 85%, 90%, 95% or 99% amino acid sequence identity over a length of at least 20 amino acids of the amino acid sequence of SEQ ID NO:9.

In certain embodiments the antibody or antigen binding fragment comprises a light chain variable domain comprising an amino acid sequence of SEQ ID NO:10, or a sequence which has at least 85%, 90%, 95% or 99% amino acid sequence identity over a length of at least 20 amino acids of the amino acid sequence of SEQ ID NO:10, and a heavy chain variable domain comprising an amino acid sequence of SEQ ID NO:11, or a sequence which has at least 85%, 90%, 95% or 99% amino acid sequence identity over a length of at least 20 amino acids of the amino acid sequence of SEQ ID NO:11. Typically, the antibody or antigen binding portion specifically binds to TLR2 and does not bind to CD32.

In certain embodiments the light chain comprises the amino acid sequence of SEQ ID NO:12, or a sequence which has at least 85%, 90%, 95% or 99% amino acid sequence identity over a length of at least 20 amino acids of the amino acid sequence of SEQ ID NO:12, and/or the heavy chain comprises the amino acid sequence of SEQ ID NO:13, or a sequence which has at least 85%, 90%, 95% or 99% amino acid sequence identity over a length of at least 20 amino acids of the amino acid sequence of SEQ ID NO:13. Typically, the antibody or antigen binding fragment is a fully humanised antibody. In certain embodiments the variable domain of the light chain and/or the variable domain of the heavy chain is joined to a constant domain derived from an antibody of the subclass immunoglobulin G, isotype 4. In certain embodiments amino acid residue 241 of a hinge region of the heavy chain is substituted from a serine residue to a proline residue. In certain embodiments the antibody or antigen binding fragment binds to mammalian Toll-like Receptor 2 with a K_{D} of 1x10⁻⁸ or less.

In certain embodiments the antibody is a murine IgG1 anti-TLR2 antibody derived from hybridoma clone T2.5 (HyCult Biotechnology b.v., Cell Sciences, Canton, USA: catalogue number 1054), or a humanised version thereof. In certain embodiments the humanised version thereof is the OPN-305 antibody, as described in W02011/003925.

In certain embodiments the TLR2 antagonist comprises a nucleic acid encoding an antibody or antigen binding fragment as described above, or a vector comprising said nucleic acid.

In certain embodiments the TLR2 antagonist is an inhibitory nucleic acid which inhibits the expression of at least one nucleic acid which encodes TLR2 protein. In certain embodiments the TLR2 antagonist is selected from the group consisting of anti-sense oligonucleotides, triple helix molecules, anti-sense DNA, anti-sense RNA, ribozyme, iRNA, miRNA, siRNA and shRNA. Accordingly, in certain embodiments the present invention provides for the administration to the subject of a therapeutically effective amount of an inhibitory nucleic acid, such as an RNAi (RNA interference) agent, for example an interfering ribonucleic acid (such as siRNA or shRNA) or a transcription template thereof, such as a DNA encoding an shRNA in order to block the expression of the TLR2 protein. In certain embodiments the inhibitory nucleic acid may be an antisense RNA molecule. Antisense causes suppression of gene expression and involves single stranded RNA fragments which physically bind to mRNA, thus blocking mRNA translation. Techniques for the preparation of inhibitory nucleic acids are known to persons skilled in the art.

The inventors have further identified that suppression of the function of TLR2 can be achieved by means of reducing the amount of ligand which is available to bind to and activate membrane bound TLR2. A reduction in the amount of ligand which is available to bind membrane bound TLR2 results in a downregulation of TLR2-mediated signalling. Accordingly, in certain embodiments the TLR2 antagonist is a soluble form of recombinant TLR2 (sTLR2), or a functional fragment thereof. The soluble form of TLR2 competes with the membrane bound form of TLR2 for TLR2 specific binding ligands. This competitive binding results in the soluble form of TLR2 effectively "mopping up" available TLR2 ligand with an associated reduction in the binding and activation of membrane bound TLR2.

In certain embodiments the soluble form of TLR2 is prepared by a recombinant technique. A soluble form of TLR2 typically comprises the extracellular domain of TLR2 only, and hence the intracellular and transmembrane domains of TLR2 as defined in Genbank Accession Number AAC 34133 (SEQ ID NO:1) are absent. In certain embodiments, the soluble form of TLR2 comprises amino acids 1 to 587 of SEQ ID NO:1. The soluble TLR2 sequence may be modified by means of the addition, deletion or substitution of one or more amino acid residues. Accordingly, in certain embodiments the soluble form of TLR2 is derived from a truncated form of the full length membrane bound TLR2 amino acid sequence or, in addition to a deletion and/or substitution of the amino acids residues relating to the intracellular and/or transmembrane domains in the membrane bound form of TLR2, a deletion and/or substitution may further be made to the amino acid residues of the extracellular domain. Any such truncation or deletion and/or substitution of the amino acid residues of the extracellular domain of the TLR2 may be made so long as the modified form of TLR2 is soluble and capable of binding a ligand which can bind to at least one epitope present on the membrane bound form of TLR2.

In certain embodiments the TLR2 antagonist, such as the soluble form of TLR2, is targeted to the pancreas or TLR2 expressed on pancreatic cells or tissue. In certain embodiments the TLR2 antagonist is targeted specifically to tumour cells. Targeting may be by any suitable means known to the person skilled in the art, such as localised delivery, the use of a delivery vector or a targeting means, such as an antibody which has binding specificity for a cell surface target expressed on pancreatic cells and/or tumour tissue. The targeting of the TLR2 antagonist in this way is advantageous as systemic administration of the TLR2 antagonist may result in global immunosuppression of the TLR2 receptor and accordingly TLR2 mediated signalling which may be undesirable in some instances. Targeting of the TLR2 antagonist, such as the soluble form TLR2, may be provided through the formation of a fusion protein, wherein said fusion protein is comprised of a TLR2 antagonist (e.g. a soluble portion of the TLR2 receptor, typically the extracellular domain or a portion thereof) conjoined to a secondary peptide, typically the Fc receptor binding protein derived from the heavy chain of an immunoglobulin, typically a human immunoglobulin. The Fc domain has been extensively used to prolong the circulatory half-life of therapeutic proteins.

In certain embodiments the TLR2 antagonist binds to the epitope present on the extracellular domain of TLR2 with an affinity constant (Ka) of at least 10⁻⁶M.

In certain embodiments the method comprises the step of administering a second TLR2 antagonist to the subject. For example, a TLR2 antagonistic antibody may be administered to prevent the activation of TLR2 and an inhibitory nucleic acid may also be administered to inhibit the expression of TLR2.

In certain embodiments the method of the invention further comprises a step of administering a therapeutically effective amount of a secondary therapeutic compound suitable for use in the treatment or prevention of pancreatic cancer. Said secondary therapeutic compound may be a chemotherapeutic agent, typically selected from the group consisting of gemcitabine, cyclophosphamide, abraxane, fluorouracil (5FU), FolFox, Folfiri and Folfirinox.

In certain embodiments the secondary therapeutic compound is gemcitabine. The inventors have surprisingly recognised that treatment with a TLR2 antagonist and gemcitabine has a synergistic effect in treating pancreatic cancer as compared with treatment with either the TLR2 antagonist or gemcitabine alone. In particular, treatment with a TLR2 antagonist plus gemcitabine synergistically increased survival and suppressed metastasis (e.g. liver metastasis) as compared with treatment with either agent alone. The treatment further enhanced an immunostimulatory response and/or reduced immunosuppressive effects, for example, the immunosuppressive effects of regulatory T cells. Furthermore, treatment with a TLR2 antagonist plus gemcitabine synergistically reduced tumour proliferation and enhanced apoptosis. Without wishing to be bound by theory, the inventors of the present invention consider that the combination of a TLR2 antagonist with gemcitabine might prove to be beneficial due to the known effect of gemcitabine in depleting myeloid derived suppressor cells, therefore contributing to an overall net outcome of immune activation when combined with a TLR2 antagonist.

In certain embodiments the secondary therapeutic compound is administered simultaneously, sequentially or separately to the TLR2 antagonist.

In certain embodiments the method of the invention further comprises a step of administering a therapeutically effective amount of a tertiary therapeutic compound suitable for use in the treatment or prevention of pancreatic cancer. Said tertiary therapeutic compound may be a chemotherapeutic agent, typically cyclophosphamide or abraxane. Cyclophosphamide has a well described ablational effect on regulatory T cells. In certain embodiments the secondary therapeutic compound is gemcitabine and the tertiary therapeutic compound is selected from the group consisting of cyclophosphamide, abraxane, fluorouracil (5FU), FolFox, Folfiri and Folfirinox, typically cyclophosphamide or abraxane. Without wishing to be bound by theory, the inventors of the present invention consider that the combination of a TLR2 antagonist with cyclophosphamide and optionally a secondary therapeutic compound such as gemcitabine might prove to be beneficial in contributing to an overall net outcome of immune activation.

In certain embodiments the tertiary therapeutic compound is administered simultaneously, sequentially or separately to the TLR2 antagonist and/or secondary therapeutic compound.

In certain embodiments, the TLR2 antagonist is administered to the subject prior to, during or following the subject undergoing a surgical procedure, such as resection. Typically the subject is suffering from pancreatic cancer. The subject may have one or more tumours. In certain embodiments the pancreatic cancer is metastatic. In alternative embodiments the pancreatic cancer is locally advanced. Alternatively, the pancreatic cancer may be localised. In certain embodiments the subject may be at risk of developing pancreatic cancer. Subjects at risk of developing pancreatic cancer may be identified, for example, using genetic testing, in particular, by testing individuals having a family history of pancreatic cancer.

Typically, the subject is a mammal, most typically a human.

In certain embodiments the TLR2 antagonist is an antagonist of human TLR2, for example the human TLR2 sequence shown in SEQ ID NO:1, or an amino acid sequence having at least 90% sequence identity thereto. In certain embodiments the TLR2 antagonist is an antagonist of murine TLR2, for example the murine TLR2 sequence shown in SEQ ID NO:2, or an amino acid sequence having at least 90% sequence identity thereto.

According to a further aspect of the invention there is provided a composition comprising a TLR2 antagonist for use in the treatment or prophylaxis of pancreatic cancer in a subject.

The embodiments described for the first aspect of the invention also apply for this aspect of the invention where applicable. In particular, the TLR2 antagonist may be a TLR2 antagonist as described above.

In certain embodiments the treatment comprises the simultaneous, separate or sequential administration of a secondary therapeutic compound. In certain embodiments the composition comprises a secondary therapeutic compound. Said secondary therapeutic compound may be a chemotherapeutic agent, typically selected from the group consisting of gemcitabine, cyclophosphamide, abraxane, fluorouracil (5FU), FolFox, Folfiri and Folfirinox.

In certain embodiments the treatment comprises the simultaneous, separate or sequential administration of a tertiary therapeutic compound. In certain embodiments the composition comprises a tertiary therapeutic compound. Said tertiary therapeutic compound may be a chemotherapeutic agent, typically cyclophosphamide or abraxane.

According to a further aspect of the invention there is provided use of a composition comprising a TLR2 antagonist in the preparation of a medicament for the treatment or prophylaxis of pancreatic cancer in a subject.

The embodiments described for the first aspect of the invention also apply for this aspect of the invention where applicable. In particular, the TLR2 antagonist may be a TLR2 antagonist as described above.

In certain embodiments, the medicament is a combined medicament comprising a secondary therapeutic compound. The secondary therapeutic compound may be a chemotherapeutic agent, typically selected from the group consisting of gemcitabine, cyclophosphamide, abraxane, fluorouracil (5FU), FolFox, Folfiri and Folfirinox. In certain embodiments the secondary therapeutic compound is gemcitabine.

In certain embodiments, the medicament further comprises a tertiary therapeutic compound, such as cyclophosphamide or abraxane.

The TLR2 antagonist may be provided in the form of a pharmaceutical composition comprising a TLR2 antagonist as described above and at least one pharmaceutically acceptable carrier, diluent, solubilizer, emulsifier, preservative and/or adjuvant. In certain embodiments, the composition includes a secondary therapeutic compound, such as gemcitabine. In certain embodiments, the composition includes a tertiary therapeutic compound, such as cyclophosphamide.

In a further aspect, the present invention extends to a screening method for the identification of compounds for use in treatment or prevention of pancreatic cancer, the method comprising the steps of:
(a) providing candidate compounds, e.g. antibodies having binding specificity for TLR2;
(b) contacting the candidate compounds with TLR2; and
(c) identifying compounds which bind to TLR2 within the region of amino acid residues His318 (H, histidine), Pro320 (P, proline), Arg321 (R, arginine) or Gln321 (Q, glutamine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of SEQ ID NO: 1 or SEQ ID NO:2;
wherein binding in this region is indicative of utility of the compound in the treatment or prevention of pancreatic cancer.

### Brief Description of the Figures

The present invention will now be described with reference to the following examples which are provided for the purpose of illustration and are not intended to be construed as being limiting on the present invention wherein:
Figure 1 shows percentage survival of mice having mean tumour diameters of 5-10mm treated with either saline ("untreated") or gemcitabine (100mg/kg, Q3dx4, i.p.) with or without TLR2 neutralising antibody (nAb) OPN-301 (T2.5). Endpoint was survival in this study;
Figure 2 shows percentage survival of mice having mean tumour diameters of 5-10mm treated with either saline ("untreated") or gemcitabine (100mg/kg, Q3dx4, i.p.) with or without TLR2 neutralising antibody OPN-305. Endpoint was survival in this study;
Figure 3 shows percentage of mice with liver metastasis following treatment of mice having mean tumour diameters of 5-10mm with either saline ("untreated") or gemcitabine (100mg/kg, Q3dx4, i.p.) with or without TLR2 nAb OPN-301 (T2.5). Liver metastasis was assessed by sectioning through the liver and counting metastasis on at least 10 slides per animals 100µm apart;
Figure 4 shows relative F4/80 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR. qPCR assays were purchased from ABI as primer-probe sets;
Figure 5 shows relative CD8a gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 6 shows relative CCR1 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 7 shows relative CCR3 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 8 shows relative NK1.1 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 9 shows relative CD208 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 10 shows relative B220 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 11 shows relative CXCR1 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 12 shows relative IL6 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 13 shows relative IL1 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 14 shows relative TNFα gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 15 shows relative hes1 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 16 shows relative hey1 gene expression following treatment of mice having mean tumour diameters of 5-10mm with either saline or gemcitabine (100mg/kg, Q3dx4, i.p.) with and without TLR2 nAb OPN-301 (T2.5). Total RNA from the pancreas of these mice was extracted and analysed by qPCR;
Figure 17 shows an assessment of tumour apoptosis and proliferation based on % BrdU+ cells following injection of mice with BrdU. Results are shown for mice untreated and mice treated with gemcitabine (100mg/kg, Q3dx4, i.p.) with and without TLR2 nAb OPN-301 (T2.5);
Figure 18 shows an assessment of tumour apoptosis and proliferation based on caspase 3 staining. Results are shown for mice untreated and mice treated with gemcitabine (100mg/kg, Q3dx4, i.p.) with and without TLR2 nAb OPN-301 (T2.5);
Figure 19 shows a proliferation index based on the results shown in Figures 17 and 18;
Figure 20 shows the number of circulating YFP+ cells per ml blood following treatment of mice with either saline or gemcitabine (100mg/kg, Q3dx4, i.p.) with and without TLR2 nAb OPN-301 (T2.5);
Figure 21 shows PDAC cells in desmoplastic stroma;
Figure 22 shows PDAC cells in desmoplastic stroma; and
Figure 23 shows PDAC cells in desmoplastic stroma.

### Detailed Description of the Invention

The present inventors have shown that a TLR2 antagonist, in particular, a TLR2 neutralising antibody, may be used to treat pancreatic cancer. In particular, percentage survival of mice was increased following treatment. Tumour progression was reduced and an increase in apoptosis was observed. Without wishing to be bound by theory, the inventors predict that treatment with a TLR2 antagonist, in particular, a TLR2 neutralising antibody, enhances an anti-tumour microenvironment. The effect observed may also involve targeting a target present on tumour cells as the present inventors have shown that TLR2 is also expressed by malignant cells. Furthermore, when the TLR2 antagonist in the form of a TLR2 neutralising antibody was combined with the secondary therapeutic compound, gemcitabine, a surprising synergistic effect was observed as compared with treatment with either agent alone. In particular, a combination of gemcitabine and TLR2 antagonist was seen to reduce liver metastasis and synergistically reduce tumour progression and increase survival.

### Definitions

As herein defined, "Toll-like Receptor 2" may be also referred to as TLR2, TLR-2 or CD282. Typically, the TLR2 is human TLR2. Alternatively, the TLR2 is murine TLR2. In further embodiments, the TLR2 is a homologue or orthologue of human TLR2 which is derived from any mammal other than a human or mouse, for example, a cow or rat. In certain embodiments the TLR2 antagonist is cross-reactive in that it mediates the suppression of TLR2 function in TLR2 derived from different species.

As herein defined, an agent is a "TLR2 antagonist" if the agent suppresses or blocks the activation or function of TLR2. The agent may inhibit or block binding of a ligand or binding compound to TLR2. This inhibition of TLR2 ligand binding may be achieved by a number of means, for example, through binding to the extracellular domain of TLR2 and partially or fully blocking the TLR2 ligand binding site, or by binding at a site other than the known TLR2 ligand binding site and inducing a conformational change upon binding which results in the TLR2 ligand binding site being altered in a manner which prevents TLR2 ligand binding or TLR2 activation. Further, the agent may inhibit or suppress intracellular signalling mediated by TLR2 following ligand binding and/or TLR2 activation. Intracellular signalling mediated following TLR2 activation and signalling results in the activation of transcription factors and the expression of genes which mediate a pro-inflammatory immune response. The agent may suppress or block TLR2 protein or gene expression, for example, by inhibiting the expression of a gene encoding a TLR2 protein.

As herein defined, the terms "blocks" and "blocking" when used in relation to TLR2 gene expression mean silencing the expression of at least one gene which results in the expression of the TLR2 protein. Gene silencing is the switching off of the expression of a gene by a mechanism other than genetic modification. Gene silencing can be mediated at the transcriptional level or at the post-transcriptional level. Transcriptional gene silencing can result in a gene being inaccessible to transcriptional machinery, and can be mediated, for example, by means of histone modifications. Post-transcriptional gene silencing results from the mRNA of a gene being destroyed, thus preventing an active gene product, such as a protein, in the present case the TLR2 protein.

The term "specifically binds" or "binding specificity" refers to the ability of a TLR2 binding compound to bind to a target epitope present on TLR2 with a greater affinity than it binds to a non-target epitope. In certain embodiments specific binding refers to binding to a particular target epitope which is present on TLR2 with an affinity which is at least 10, 50, 100, 250, 500, or 1000 times greater than the affinity for a non-target epitope. Binding affinity may be determined by an affinity ELISA assay, a BIAcore assay, a kinetic method or by an equilibrium/solution method.

As herein defined, an "epitope" refers to a plurality of amino acid residues which are capable of being recognised by, and bound to by, a binding compound, such as a ligand, small molecule or antibody. Epitopes are generally comprised of chemically active surface groups and have specific three-dimensional structural characteristics, as well as specific charge characteristics which contribute to the three-dimensional structure of the epitope. The TLR2 antagonist antagonises the functional activity of TLR2 and as such binds to an epitope known as an inhibiting epitope or an inhibitory epitope. An "inhibiting" or "inhibitory" epitope means an epitope present on TLR2 that, when bound by a binding compound such as a small molecule or an antibody, results in the loss of biological activity of TLR2, for example due to the binding compound preventing the binding of TLR2 by a TLR2 agonist. The epitope that is present on TLR2, and which is bound by the binding compounds in order to antagonise TLR2 function, may comprise five or more amino acid residues.

The TLR2 antagonist of the invention binds to a non-contiguous epitope. A "non-contiguous epitope" is an epitope that is comprised of a series of amino acid residues that are non-linear in alignment, such that the residues are spaced or grouped in a non-continuous manner along the length of a polypeptide sequence. The non-contiguous epitope described herein is a discontinuous scattered epitope wherein the residues which contribute to the epitope are provided in three or more groups of linear amino acid sequences arranged along the length of the TLR2 polypeptide.

As used herein, the term "subject" refers to an animal, preferably a mammal and in particular a human.

The term "consisting essentially of" as used herein means that the invention necessarily includes the listed items and is open to including unlisted items that do not materially affect the basic and novel properties of the invention.

The nomenclature used to describe the polypeptide constituents of the present invention follows the conventional practice wherein the amino group (N) is presented to the left and the carboxyl group to the right of each amino acid residue.

The expression "amino acid" as used herein is intended to include both natural and synthetic amino acids, and both D and L amino acids. A synthetic amino acid also encompasses chemically modified amino acids, including, but not limited to, salts, and amino acid derivatives such as amides. Amino acids can be modified by methylation, amidation, acetylation or substitution with other chemical groups which can change the circulating half life without adversely affecting their biological activity.

The terms "peptide", "polypeptide" and "protein" are used herein interchangeably to describe a series of at least two amino acids covalently linked by peptide bonds or modified peptide bonds such as isosteres. No limitation is placed on the maximum number of amino acids which may comprise a peptide or protein. Furthermore, the term polypeptide extends to fragments, analogues and derivatives of a peptide, wherein said fragment, analogue or derivative retains the same biological functional activity as the peptide from which the fragment, derivative or analogue is derived

Furthermore the term "fusion protein" as used herein can also be taken to mean a fusion polypeptide, fusion peptide or the like, or may also be referred to as an immunoconjugate. The term "fusion protein" refers to a molecule in which two or more subunit molecules, typically polypeptides, are covalently or non-covalently linked.

As used herein, the term "therapeutically effective amount" means an amount which is sufficient to show benefit to the subject to whom the composition or TLR2 antagonist is administered, e.g. by reducing the severity of at least one symptom of pancreatic cancer, by reducing tumour size, by suppressing tumour growth, by inhibiting angiogenesis, by suppressing metastasis, by suppressing a T regulatory cell response and/or by promoting a Th1 response.

As used herein, the term "treatment" and associated terms such as "treat" and "treating" means the reduction of the progression or severity of pancreatic cancer or at least one symptom thereof, e.g. by reducing tumour size, by suppressing tumour growth, by inhibiting angiogenesis, by suppressing metastasis, by suppressing a T regulatory cell response and/or by promoting a Th1 response. The term "treatment" refers to any regimen that can benefit a subject. The treatment may be in respect of an existing pancreatic cancer condition or may be prophylactic (preventative treatment). Treatment may include curative, alleviative or prophylactic effects. References herein to "therapeutic" and "prophylactic" treatments are to be considered in their broadest context. The term "therapeutic" does not necessarily imply that the subject is treated until total recovery. Similarly, "prophylactic" does not necessarily mean that the subject will not eventually contract a disease condition.

Unless otherwise defined, all technical and scientific terms used herein have the meaning commonly understood by a person who is skilled in the art in the field of the present invention.

Throughout the specification, unless the context demands otherwise, the terms "comprise" or "include", or variations such as "comprises" or "comprising", "includes" or "including" will be understood to imply the inclusion of a stated integer or group of integers, but not the exclusion of any other integer or group of integers.

As used herein, terms such as "a", "an" and "the" include singular and plural referents unless the context clearly demands otherwise. Thus, for example, reference to "an active agent" or "a pharmacologically active agent" includes a single active agent as well as two or more different active agents in combination, while references to "a carrier" includes mixtures of two or more carriers as well as a single carrier, and the like.

### Antibodies

The TLR2 antagonist may be an antibody or an antigen binding fragment thereof. An "antibody" is an immunoglobulin, whether natural or partly or wholly synthetically produced. The term also covers any polypeptide, protein or peptide having a binding domain that is, or is homologous to, an antibody binding domain. These can be derived from natural sources, or they may be partly or wholly synthetically produced. Examples of antibodies are the immunoglobulin isotypes and their isotypic subclasses and fragments which comprise an antigen binding domain such as Fab, scFv, Fv, dAb or Fd, and a bi-specific antibody.

In certain embodiments the antibody may be a camelid antibody, in particular a camelid heavy chain antibody. Further, the antibody fragment may be a domain antibody or a nanobody derived from a camelid heavy chain antibody. In certain embodiments the antibody may be a shark antibody or a shark derived antibody.

In certain embodiments the antibody is an "isolated antibody", this meaning that the antibody is (1) free of at least some proteins with which it would normally be found, (2) is essentially free of other proteins from the same source, e.g., from the same species, (3) is expressed by a cell from a different species, or (4) does not occur in nature.

As antibodies can be modified in a number of ways, the term "antibody" should be construed as covering any binding member or substance having a binding domain with the required specificity. The antibody of the invention may be a monoclonal antibody, or a fragment, derivative, functional equivalent or homologue thereof. The term includes any polypeptide comprising an immunoglobulin binding domain, whether natural or wholly or partially synthetic. Chimeric molecules comprising an immunoglobulin binding domain, or equivalent, fused to another polypeptide are therefore included.

The constant region of the antibody may be of any suitable immunoglobulin subtype. However, it is preferred that the antibody subtype is IgG1. In alternative embodiments the subtype of the antibody may be of the class IgA, IgM, IgD and IgE where a human immunoglobulin molecule is used. Such an antibody may further belong to any subclass e.g. IgG1, IgG2a, IgG2b, IgG3 and IgG4.

Fragments of a whole antibody can perform the function of antigen binding. Examples of such binding fragments are a Fab fragment comprising of the VL, VH, CL and CH1 antibody domains; an Fv fragment consisting of the VL and VH domains of a single antibody; a F(ab')2 fragment; a bivalent fragment comprising two linked Fab fragments; a single chain Fv molecule (scFv), wherein a VH domain and a VL domain are linked by a peptide linker which allows the two domains to associate to form an antigen binding site; and a bi-specific antibody, which may be multivalent or multispecific fragments constructed by gene fusion.

A fragment of an antibody or of a polypeptide for use in the present invention, for example, a fragment of a TLR2 specific antibody, generally means a stretch of amino acid residues of at least 5 to 7 contiguous amino acids, often at least about 7 to 9 contiguous amino acids, typically at least about 9 to 13 contiguous amino acids, more preferably at least about 20 to 30 or more contiguous amino acids and most preferably at least about 30 to 40 or more consecutive amino acids.

A "derivative" of such an antibody or polypeptide, or of a fragment of a TLR2 specific antibody means an antibody or polypeptide modified by varying the amino acid sequence of the protein, e.g. by manipulation of the nucleic acid encoding the protein or by altering the protein itself. Such derivatives of the natural amino acid sequence may involve insertion, addition, deletion and/or substitution of one or more amino acids, preferably while providing a peptide having TLR2 binding activity. Preferably such derivatives involve the insertion, addition, deletion and/or substitution of 25 or fewer amino acids, more preferably of 15 or fewer, even more preferably of 10 or fewer, more preferably still of 4 or fewer and most preferably of 1 or 2 amino acids only.

In certain embodiments humanized antibodies are also provided. A humanised antibody may be a modified antibody having the hypervariable region of a TLR2 specific antibody and the constant region of a human antibody. Thus the binding member may comprise a human constant region. The variable region other than the hypervariable region may also be derived from the variable region of a human antibody and/or may also be derived from a TLR2 specific antibody. In other cases, the entire variable region may be derived from a murine monoclonal TLR2 specific antibody and the antibody is said to be chimerised.

It is possible to take monoclonal and other antibodies and use techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. A hybridoma or other cell producing an antibody may be subject to genetic mutation or other changes, which may or may not alter the binding specificity of antibodies produced.

In certain embodiments the therapeutically effective amount comprises the antibody in a range chosen from 1 µg/kg to 20 mg/kg, 1 g/kg to 10 mg/kg, 1 µg/kg to 1 mg/kg, 10 µg/kg to 1 mg/kg, 10 µg/kg to 100 pg/kg and 500 pg/kg to 1 mg/kg.

### Production of Antibodies

The antibodies provided for use in the present invention may be provided by a number of techniques. For example, a combinatorial screening technique such as a phage display-based biopanning assay may be used to in order to identify amino acid sequences which have binding specificity to the binding epitopes described above. Such phage display biopanning techniques involve the use of phage display libraries, which are utilised in methods which identify suitable epitope binding ligands in a procedure which mimics immune selection, through the display of antibody binding fragments on the surface of filamentous bacteria. Phage with specific binding activity are selected. The selected phage can thereafter be used in the production of chimeric, CDR-grafted, humanised or human antibodies.

In further embodiments the antibody is a monoclonal antibody, which may be produced using any suitable method which produces antibody molecules by continuous cell lines in culture. Chimeric antibodies or CDR-grafted antibodies are further provided for use in the present invention. In certain embodiment, the antibodies for use in the invention may be produced by the expression of recombinant DNA in host cell.

In certain embodiments the monoclonal antibodies may be human antibodies, produced using transgenic animals, for example, transgenic mice, which have been genetically modified to delete or suppress the expression of endogenous murine immunoglobulin genes, with loci encoding for human heavy and light chains being expressed in preference, this resulting in the production of fully human antibodies.

In certain embodiments the TLR2 antagonist is a binding fragment which is derived from an antibody, for example, an antibody binding fragment, such as a Fab, F(ab')2, Fv or a single chain Fv (scFV).

In certain embodiments the TLR2 antagonist comprises a polyclonal antibody, a chimeric antibody, a synthesized or synthetic antibody, a fusion protein or fragment thereof, a natural or synthetic chemical compound or a peptidomimetic.

The antibodies or antigen fragments for use in the present invention may also be generated wholly or partly by chemical synthesis. The antibodies can be readily prepared according to well-established, standard liquid or, preferably, solid-phase peptide synthesis methods, general descriptions of which are broadly available and are well known by the person skilled in the art. Further, they may be prepared in solution, by the liquid phase method or by any combination of solid-phase, liquid phase and solution chemistry.

Another convenient way of producing antibodies or antibody fragments suitable for use in the present invention is to express nucleic acid encoding them by use of nucleic acid in an expression system.

Nucleic acid for use in accordance with the present invention may comprise DNA or RNA and may be wholly or partially synthetic. In a preferred aspect, nucleic acid for use in the invention codes for antibodies or antibody fragments of the invention as defined above. The skilled person will be able to determine substitutions, deletions and/or additions to such nucleic acids which will still provide an antibody or antibody fragment of the present invention.

Nucleic acid sequences encoding antibodies or antibody fragments for use with the present invention can be readily prepared by the skilled person. These techniques include (i) the use of the polymerase chain reaction (PCR) to amplify samples of such nucleic acid, e.g. from genomic sources, (ii) chemical synthesis, or (iii) preparing cDNA sequences. DNA encoding antibody fragments may be generated and used in any suitable way known to those of skill in the art, including by taking encoding DNA, identifying suitable restriction enzyme recognition sites either side of the portion to be expressed, and cutting out said portion from the DNA. The portion may then be operably linked to a suitable promoter in a standard commercially available expression system. Another recombinant approach is to amplify the relevant portion of the DNA with suitable PCR primers. Modifications to the sequences can be made, e.g. using site directed mutagenesis, to lead to the expression of modified peptide or to take account of codon preferences in the host cells used to express the nucleic acid.

The nucleic acid may be comprised as constructs in the form of a plasmid, vector, transcription or expression cassette which comprises at least one nucleic acid as described above. The construct may be comprised within a recombinant host cell which comprises one or more constructs as above. Expression may conveniently be achieved by culturing under appropriate conditions recombinant host cells containing the nucleic acid. Following production by expression the antibody or antibody fragments may be isolated and/or purified using any suitable technique, then used as appropriate.

Systems for cloning and expression of a polypeptide in a variety of different host cells are well known. Suitable host cells include bacteria, mammalian cells, yeast, insect and baculovirus systems. Mammalian cell lines available in the art for expression of a heterologous polypeptide include Chinese hamster ovary (CHO) cells, HeLa cells, baby hamster kidney cells, NS0 mouse myeloma cells. A common, preferred bacterial host is *E*. *coli.* The expression of antibodies and antibody fragments in prokaryotic cells such as *E*. *coli* is well established in the art. Expression in eukaryotic cells in culture is also available to those skilled in the art as an option for production of a binding member. General techniques for the production of antibodies are well known to the person skilled in the field.

In certain embodiments of the invention, recombinant nucleic acids comprising an insert coding for a heavy chain variable domain and/or for a light chain variable domain of antibodies are provided. By definition such nucleic acids comprise coding single stranded nucleic acids, double stranded nucleic acids consisting of said coding nucleic acids and of complementary nucleic acids thereto, or these complementary (single stranded) nucleic acids themselves.

Furthermore, nucleic acids encoding a heavy chain variable domain and/or a light chain variable domain of antibodies can be enzymatically or chemically synthesised nucleic acids having the authentic sequence coding for a naturally-occurring heavy chain variable domain and/or for the light chain variable domain, or a mutant thereof.

Recombinant DNA technology may be used to improve the antibodies of the invention. Thus, chimeric antibodies may be constructed in order to decrease the immunogenicity thereof in diagnostic or therapeutic applications. Moreover, immunogenicity within, for example, a transgenic organism such as a pig, may be minimised, by altering the antibodies by CDR grafting in a technique analogous to humanising antibodies. In order to reduce immunogenicity within a human recipient, the invention may employ recombinant nucleic acids comprising an insert coding for a heavy chain variable domain of an antibody fused to a human constant domain. Likewise the invention concerns recombinant DNAs comprising an insert coding for a light chain variable domain of an antibody fused to a human constant domain kappa or lambda region.

Antibodies may moreover be generated by mutagenesis of antibody genes to produce 5 artificial repertoires of antibodies. This technique allows the preparation of antibody libraries. Antibody libraries are also available commercially. Hence, the present invention advantageously employs artificial repertoires of immunoglobulins, preferably artificial scFv repertoires, as an immunoglobulin source in order to identify binding molecules which have specificity for the epitope described above.

### Antibody selection systems

Immunoglobulins which are able to bind to the epitope of the present invention and which accordingly may be used in the methods of the invention can be identified using any technique known to the skilled person. Such immunoglobulins may be conveniently isolated from libraries comprising artificial repertoires of immunoglobulin polypeptides. A "repertoire" refers to a set of molecules generated by random, semi-random or directed variation of one or more template molecules, at the nucleic acid level, in order to provide a multiplicity of binding specificities. Methods for generating repertoires are well characterised in the art.

Any library selection system may be used in conjunction with the invention. Selection protocols for isolating desired members of large libraries are known in the art, as typified by phage display techniques. Such systems, in which diverse peptide sequences are displayed on the surface of filamentous bacteriophage, have proven useful for creating libraries of antibody fragments (and the nucleotide sequences that encode them) for the *in-vitro* selection and amplification of specific antibody fragments that bind a target antigen. The nucleotide sequences encoding the VH and VL regions are linked to gene fragments which encode leader signals that direct them to the periplasmic space of *E*. *coli* and as a result the resultant antibody fragments are displayed on the surface of the bacteriophage, typically as fusions to bacteriophage coat proteins (for example pIII or pVIII). Alternatively, antibody fragments are displayed externally on lambda phage capsids (phage bodies). An advantage of phage-based display systems is that, because they are biological systems, selected library members can be amplified simply by growing the phage containing the selected library member in bacterial cells. Furthermore, since the nucleotide sequence that encodes the polypeptide library member is contained on a phage or phagemid vector, sequencing, expression and subsequent genetic manipulation is relatively straight forward.

Methods for the construction of bacteriophage antibody display libraries and lambda phage expression libraries are well known in the art.

An alternative to the use of phage or other cloned libraries is to use nucleic acid, preferably RNA, derived from the B cells of an animal which has been immunised with the selected target, e.g. the TLR2 epitope described above.

Isolation of V-region and C-region mRNA permits antibody fragments, such as Fab or Fv, to be expressed intracellularly. Briefly, RNA is isolated from the B cells of an immunised animal, for example from the spleen of an immunised mouse or the circulating B cells of a llama, and PCR primers used to amplify VH and VL cDNA selectively from the RNA pool. The VH and VL sequences thus obtained are joined to make scFv antibodies. PCR primer sequences may be based on published VH and VL sequences.

### Peptidomimetics

Peptide analogues, such as peptidomimetics or peptide mimetics are non-peptide compounds with properties representative of a template peptide. Such peptide analogues are typically developed using computerised molecular modelling. Peptidomimetics which are structurally similar to peptides which have affinity and binding specificity to the TLR2 binding epitope described above may be used to mediate similar diagnostic, prophylactic and therapeutic effects.

Peptidomimetics are typically structurally similar to a template peptide, but have one or more peptide linkages replaced by an alternative linkage, by methods which are well known in the art. For example, a peptide which has a binding specificity for the TLR2 epitope described above may be modified such that it comprises amide bond replacement, incorporation of non peptide moieties, or backbone cyclisation. Suitably if cysteine is present the thiol of this residue is capped to prevent damage of the free sulphate group. A peptide may further be modified from the natural sequence to protect the peptides from protease attack.

Suitably a peptide of and for use in the present invention may be further modified using at least one of C and/or N-terminal capping, and/or cysteine residue capping. Suitably, a peptide of and for use in the present invention may be capped at the N terminal residue with an acetyl group. Suitably, a peptide of and for use in the present invention may be capped at the C terminal with an amide group. Suitably, the thiol groups of cysteines are capped with acetamido methyl groups.

Expression, isolation and purification of polypeptides defining the epitope of the invention and fragments thereof may be accomplished by any suitable technique. A method for producing polypeptides comprises culturing host cells transformed with a recombinant expression vector encoding a polypeptide under conditions that promote expression of the polypeptide, then recovering the expressed polypeptides from the culture. The person skilled in the art will recognise that the procedure for purifying the expressed polypeptides will vary according to such factors as the type of host cells employed, and whether the polypeptide is intracellular, membrane-bound or a soluble form that is secreted from the host cell.

Any suitable expression system may be employed. The vectors include a DNA encoding a polypeptide or fragment of the invention, operably linked to suitable transcriptional or translational regulatory nucleotide sequences, such as those derived from a mammalian, avian, microbial, viral, bacterial, or insect gene. Nucleotide sequences are operably linked when the regulatory sequence functionally relates to the DNA sequence. Thus, a promoter nucleotide sequence is operably linked to a DNA sequence if the promoter nucleotide sequence controls the transcription of the DNA sequence. An origin of replication that confers the ability to replicate in the desired (*E.coli*) host cells, and a selection gene by which transformants are identified, are generally incorporated into the expression vector. In addition, a sequence encoding an appropriate signal peptide (native or heterologous) can be incorporated into expression vectors. A DNA sequence for a signal peptide (secretory leader) may be fused in frame to the nucleic acid sequence of the invention so that the DNA is initially transcribed, and the mRNA translated, into a fusion protein comprising the signal peptide. A signal peptide that is functional in the intended host cells promotes extracellular secretion of the polypeptide. The signal peptide is cleaved from the polypeptide during translation, but allows secretion of polypeptide from the cell.

Suitable host cells for expression of polypeptides include higher eukaryotic cells and yeast. Prokaryotic systems are also suitable. Mammalian cells, and in particular CHO cells are particularly preferred for use as host cells.

### Small Molecules

A substance identified as an antagonist of the TLR2 receptor may be a peptide or may be non-peptide in nature, for example a peptidomimetic as described hereinbefore. However, non-peptide "small molecules" are often preferred for many *in-vivo* pharmaceutical uses. Accordingly, a mimetic or mimic of a TLR2 binding compound may be designed for use in the present invention.

The designing of mimetics to a known pharmaceutically active compound is a known approach to the development of pharmaceuticals based on a "lead" compound. This might be desirable where the active compound is difficult or expensive to synthesise, or where it is unsuitable for a particular method of administration. For example, peptides are not well suited as active agents for oral compositions and administration as they are degraded by proteases present in the alimentary canal. Mimetic design, synthesis and testing may be used to avoid randomly screening large number of molecules for a target property.

There are several steps commonly taken in the design of a mimetic from a compound having a given target property. Firstly, the particular parts of the compound that are critical and/or important in determining the target property are determined. In the case of a peptide, this can be done by systematically varying the amino acid residues in the peptide, for example by substituting each amino acid residue in turn. These parts or residues constituting the active region of the compound are known as its "pharmacophore".

Once the pharmacophore has been determined, its structure is modelled according to its physical properties, e.g. stereochemistry, bonding, size and/or charge, using data from a range of sources, e.g. spectroscopic techniques, X-ray diffraction data and NMR. Computational analysis, similarity mapping (which models the charge and/or volume of a pharmacophore, rather than the bonding between atoms) and other techniques can also be used in this modelling process.

In a variant of this approach, the three-dimensional structure of the TLR2 binding compound is modelled. This can be especially useful where the ligand and/or binding partner change conformation on binding, allowing the model to take account of the design of the mimetic.

A template molecule is then selected onto which chemical groups which mimic the pharmacophore can be grafted. The template molecule and the chemical groups grafted on to it can conveniently be selected so that the mimetic is easy to synthesise, is likely to be pharmacologically acceptable, and does not degrade *in-vivo,* while retaining the biological activity of the lead compound. The mimetic or mimetics found by this approach can then be screened to see whether they have the target property, or to what extent they exhibit it. Further optimisation or modification can then be carried out to arrive at one or more final mimetics for *in-vivo* or clinical testing.

The mimetic binding compound may be a natural or synthetic chemical compound used in drug screening programmes. Extracts of plants which contain several characterised or uncharacterised components may also be used.

Combinatorial library technology provides an efficient way of testing a potentially vast number of different substances for ability their ability to bind to an epitope or to modulate the activity of a ligand which binds to an epitope. Prior to, or as well as, being screened for modulation of activity, test substances may be screened for ability to interact with the polypeptide, e.g. in a yeast two-hybrid system (which requires that both the polypeptide and the test substance can be expressed in yeast from encoding nucleic acid). This may be used as a coarse screen prior to testing a substance for actual ability to modulate activity of the polypeptide.

The amount of test substance or compound which may be added to an assay of the invention will normally be determined by trial and error depending upon the type of compound used. Typically, from about 0.01 to 100 nM concentrations of putative inhibitor compound may be used, for example from 0.1 to 10 nM. Greater concentrations may be used when a peptide is the test substance.

### Administration

The TLR2 antagonist may be administered alone, but will preferably be administered as a pharmaceutical composition, which will generally comprise a suitable pharmaceutically acceptable excipient, diluent or carrier selected depending on the intended route of administration. Examples of suitable pharmaceutical carriers include water, glycerol, ethanol and other GRAS reagents.

The TLR2 antagonist may be administered to a subject in need of treatment via any suitable route. As detailed herein, it is preferred that the composition is administered parenterally by injection or infusion. Examples of preferred routes for parenteral administration include, but are not limited to, intravenous, intracardial, intraarterial, intraperitoneal, intramuscular, intracavity, subcutaneous, transmucosal, inhalation and transdermal. Routes of administration may further include topical and enteral, for example, mucosal (including pulmonary), oral, nasal, rectal.

The composition may be delivered as an injectable composition. For intravenous, intramuscular, intradermal or subcutaneous application, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as sodium chloride injection, Ringer's injection or, Lactated Ringer's injection. Preservatives, stabilisers, buffers, antioxidants and/or other additives may be included, as required.

The composition may also be administered via microspheres, liposomes, other microparticulate delivery systems or sustained release formulations placed in certain tissues including blood.

The actual dose administered, and rate and time-course of administration, will depend on, and can be determined with due reference to, the nature and severity of the condition which is being treated, as well as factors such as the age, sex and weight of the subject to be treated and the route of administration. Further due consideration should be given to the properties of the composition, for example, its binding activity and in-vivo plasma life, the concentration of the antagonist in the formulation, as well as the route, site and rate of delivery.

Dosage regimens can include a single administration of the composition of the invention, or multiple administrative doses of the composition. The compositions can further be administered sequentially or separately with other therapeutics and medicaments which are used for the treatment of pancreatic cancer.

The composition may be administered as a single dose or as repeated doses. Examples of dosage regimens which can be administered to a subject can be selected from the group comprising, but not limited to, 11µg/kg/day through to 20mg/kg/day, 11µg/kg/day through to 10mg/kg/day, 10µg/kg/day through to 1mg/kg/day.

The TLR2 antagonist may be orally administered to the subject, for example, at a dose of from about 1 mg/kg to about 10 mg/kg of the subject's body weight per day. In certain embodiments the dose of the TLR2 antagonist is from about 100 mg per day to about 1000 mg per day. In certain further embodiments the dose of the TLR2 antagonist is from about 200 mg per day to about 300 mg per day. In certain embodiments the TLR2 antagonist is administered to the subject parenterally with a dosage range of between about 0.001 mg/kg to 1.0 mg/kg of the subject's body weight. Typically, the TLR2 antagonist is administered to the subject for a time and under conditions sufficient to down regulate the level and/or activity of TLR2.

### Candidate Compounds

The present invention provides an assay method for identifying compounds for use in the treatment or prevention of pancreatic cancer. The method comprises identifying compounds which bind to the same epitope on TLR2 as that bound by the T2.5 and OPN-305 antibodies.

In certain embodiments the candidate compound is selected from the group consisting of a peptide, for example an antibody or antibody fragment, a chemical compound and a peptidomimetic. In certain embodiments the candidate compound is a natural or synthetic chemical compound used in a drug screening programmes. Extracts of plants which contain several characterised or uncharacterised components may be used.

The precise format of the candidate compound screening assay may be varied by those skilled in the art using routine skill and knowledge. Combinatorial library technology provides an efficient way of testing a potentially vast number of different substances for the ability to bind to an epitope. The amount of candidate compound which may be added to an assay will normally be determined by trial and error depending upon the type of compound used. Typically, from about 0.01 to 100 nM concentrations of the candidate compound may be used, for example from 0.1 to 10 nM. Greater concentrations may be used when the candidate binding compound is a peptide. A candidate compound which has affinity and binding specificity for the binding epitope described herein may be isolated and/or purified, manufactured and/or used to modulate TLR2 functional activity in the treatment or prevention of pancreatic cancer.

### Sequence Identity

The present invention extends to the use of sequences having at least 80%, 85%, 90%, 95%, 97%, 98% or 99% amino acid sequence identity to the sequences of the TLR2 antagonists described herein. Such sequences or polypeptides may comprise a sequence which is substantially homologous to a polypeptide having the amino acid sequence of a TLR2 antagonist described herein, but may have a different amino acid sequence because of one or more deletions, insertions, or substitutions. The substantially homologous polypeptide may include 1, 2 or more amino acid alterations. Alternatively, or in addition, the substantially homologous polypeptide may consist of a truncated version of a TLR2 antagonist described herein which has been truncated by 1, 2 or more amino acids. In certain embodiments sequence identity is over a length of at least 20, 25, 30, 35 or 40 amino acids of the amino acid sequence in question. In certain embodiments sequence identity is over the complete length of the amino acid sequence in question (e.g. any one of SEQ ID NO:8, 9, 10, 11, 12 and/or 13).

A given amino acid may be replaced, for example, by a residue having similar physiochemical characteristics. Examples of such conservative substitutions include substitution of one aliphatic residue for another, such as lle, Val, Leu, or Ala for one another; substitutions of one polar residue for another, such as between Lys and Arg, Glu and Asp, or GIn and Asn; or substitutions of one aromatic residue for another, such as Phe, Trp, or Tyr for one another. Other conservative substitutions, e.g., involving substitutions of entire regions having similar hydrophobicity characteristics, are well known.

Similarly, the nucleic acids for use herein may be variants that differ from a native DNA sequence because of one or more deletions, insertions or substitutions, but that encode a biologically active polypeptide.

As used herein, percentage amino acid sequence identity may be determined, using any method known to the person skilled in the art, for example, by comparing sequence information using the GAP computer program, version 6.0 described by Devereux et al. (Nucl. Acids Res. 12:387, 1984) and available from the University of Wisconsin Genetics Computer Group (UWGCG).

The present invention will now be described with reference to the following examples which are provided for the purpose of illustration and are not intended to be construed as being limiting on the present invention.

### EXAMPLES

### Mouse Model

The genetic model of pancreatic cancer used herein accurately recapitulates all stages of the human disease and there is a prominent immunosuppressive leukocyte infiltrate, even in pre-invasive lesions, of tumour associated macrophages (TAM), MDSC and Tregs, that persists through invasive cancer. The model was generated by targeting endogenous KrasG12D to progenitor cells of the mouse pancreas and recapitulating a full spectrum of pancreatic intraepithelial neoplasias (PanINs) (KC mice = LSL-KRASG12D/+, PDX-1-Cre) that progress at low frequency to invasive and metastatic tumours. At 10 weeks of age, mice in which endogenous KrasG12D is targeted to pancreatic progenitor cells have a spectrum of pre-invasive lesions (PanIN 1, further progression to PanIN 2a,b and 3) with a marked inflammatory component that includes innate and adaptive immune cells. When a point mutant allele of the Li-Fraumeni human ortholog Trp53R172H is concomitantly targeted to the pancreas, all mice develop invasive and metastatic disease (KPC mice = LSL-KrasG12D/+;LSL-Trp53R172H/+;Pdx-1-Cre). Cell lines, KPC cell lines (kras-p53-cancer cell lines derived from LSL-KrasG12D/+;LSL-Trp53R172H/+;Pdx-1-Cre tumour bearing mice) have been generated and grow orthotopically in C57B1/6 mice. The present inventors recently established a tamoxifen inducible PDX-1-Cre (PDX-1-CreERT; generate i-KPC mice) and also hold the Elastase-CreERT in their group (to target the pancreatic azinar cells). All mouse models are established on a C57B1/6 background.

### Objectives

To study the therapeutic potential of TLR2 antagonists with and without the combination of conventional chemotherapy in a genetic model of pancreatic cancer looking at:
1. tumour development, progression and survival;
2. leukocyte infiltrate, function and phenotype;
3. angiogenic switch and vascular editing; and
4. interaction between innate and adaptive immunity in the microenvironment.

### Research outline

Research was carried out using the genetic model of pancreatic cancer (LSL-KrasG12D/+;LSL-Trp53R172H/+).

The group size was calculated on the basis to obtain a 90% chance of detecting a statistically significant difference given a 50% response (i.e. 50% tumour growth/incidence or metastasis or histological score, or a 50% difference in macrophage recruitment, difference in macrophage phenotype).

For the following experiments, up to n=6 LSL-KrasG12D/+;LSL-Trp53R172H/+;Pdx-1-Cre female mice/group were recruited.

In addition to the combination with the standard of care, gemcitabine, the present inventors will test the combination of TLR2 nAb with a combinational chemotherapy regime of gemcitabine and low dose cyclophosphamide to enhance an anti-tumour microenvironment at a later time point pending the data generated.

### Method

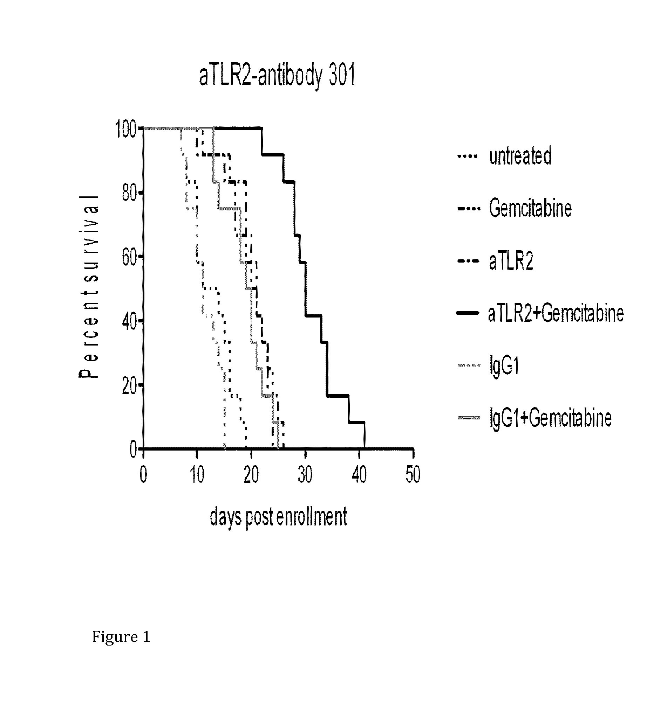

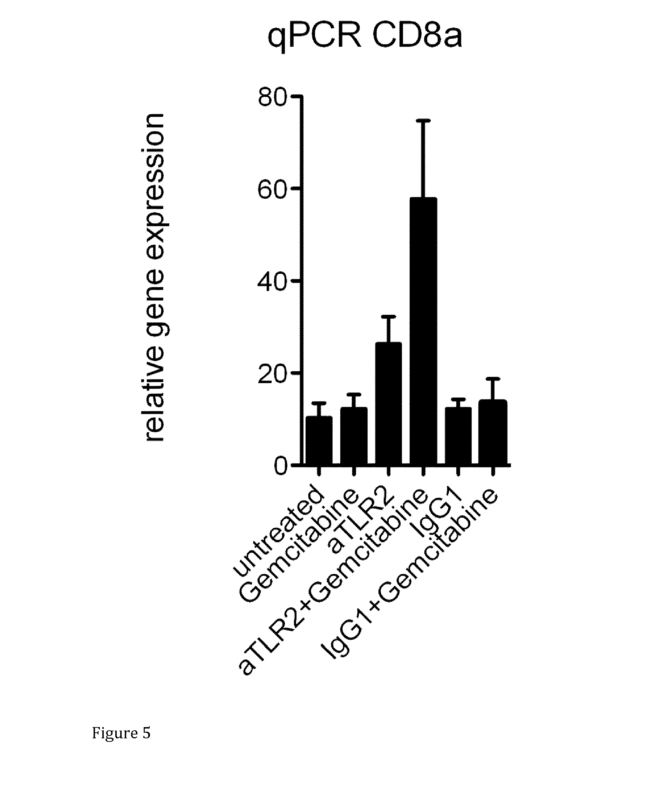

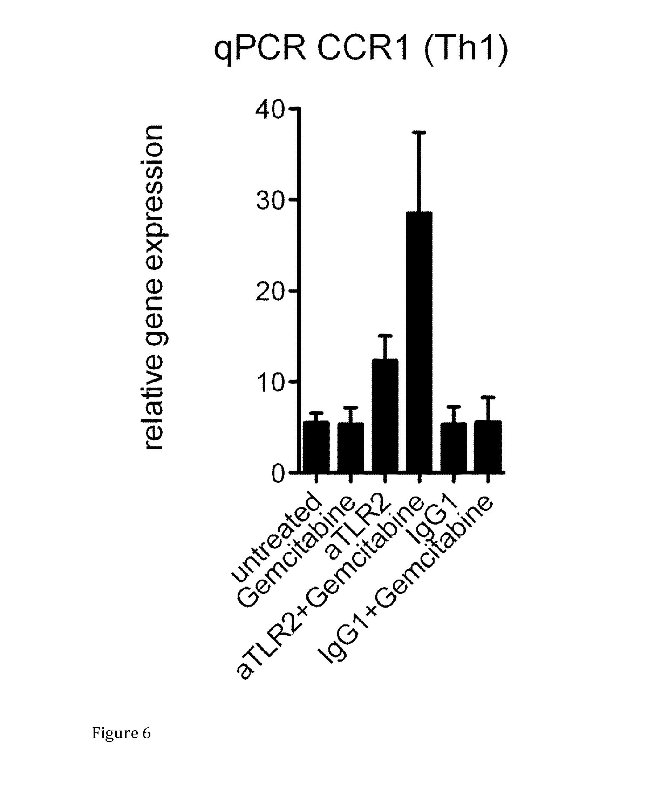

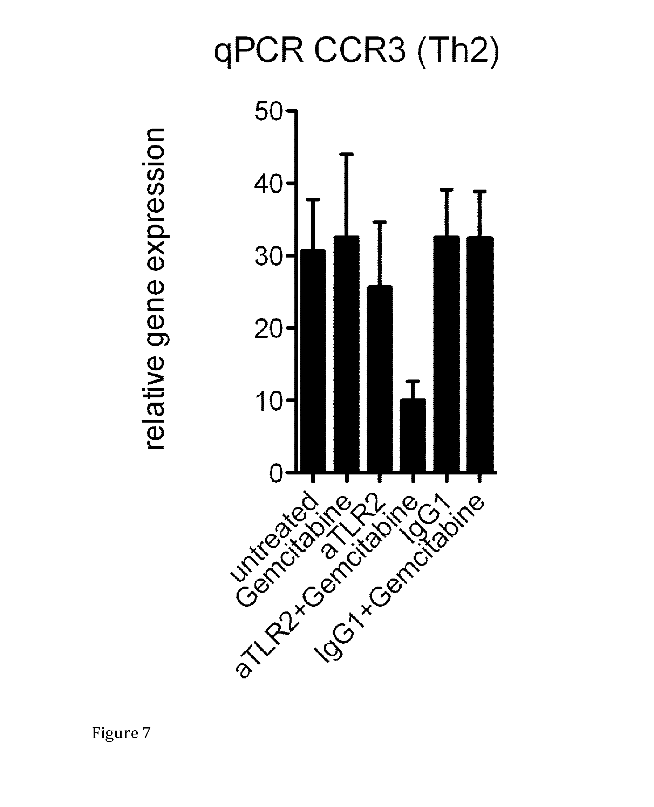

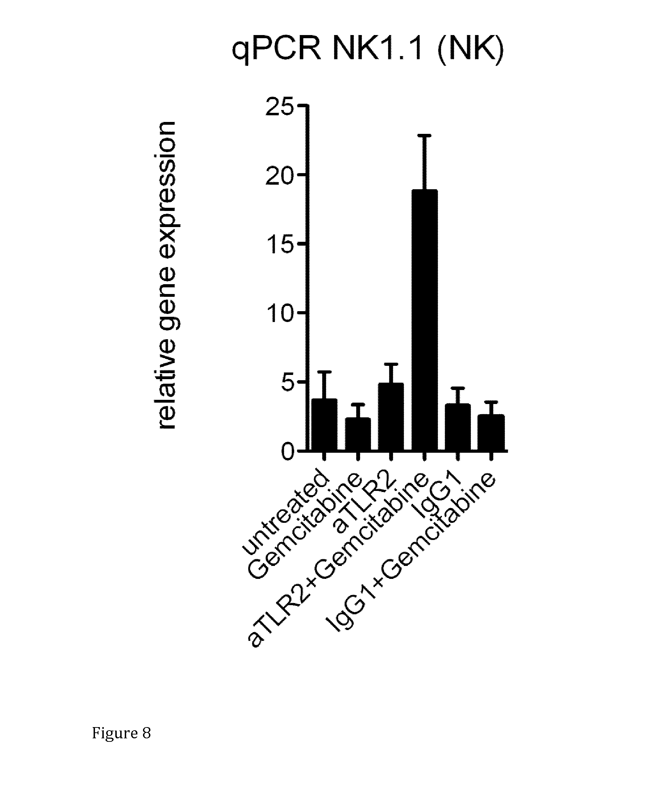

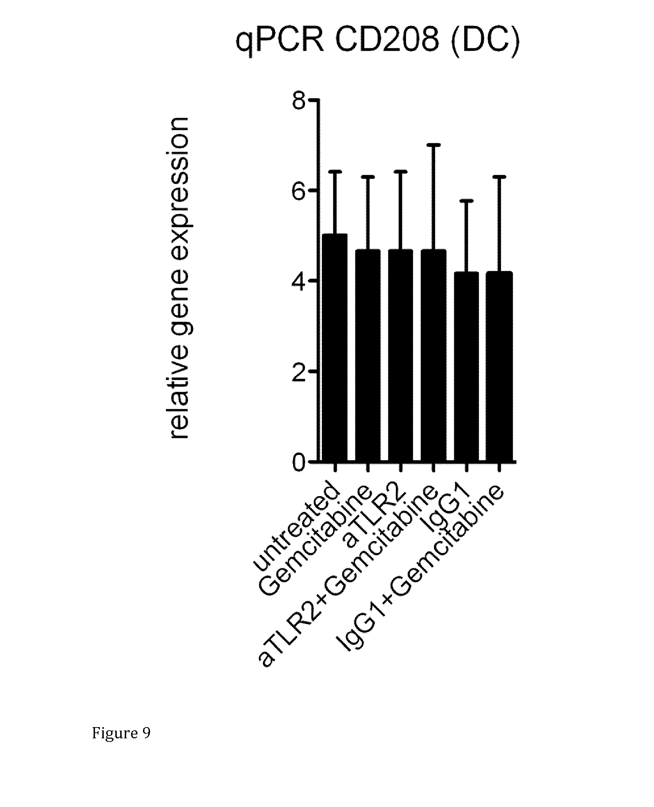

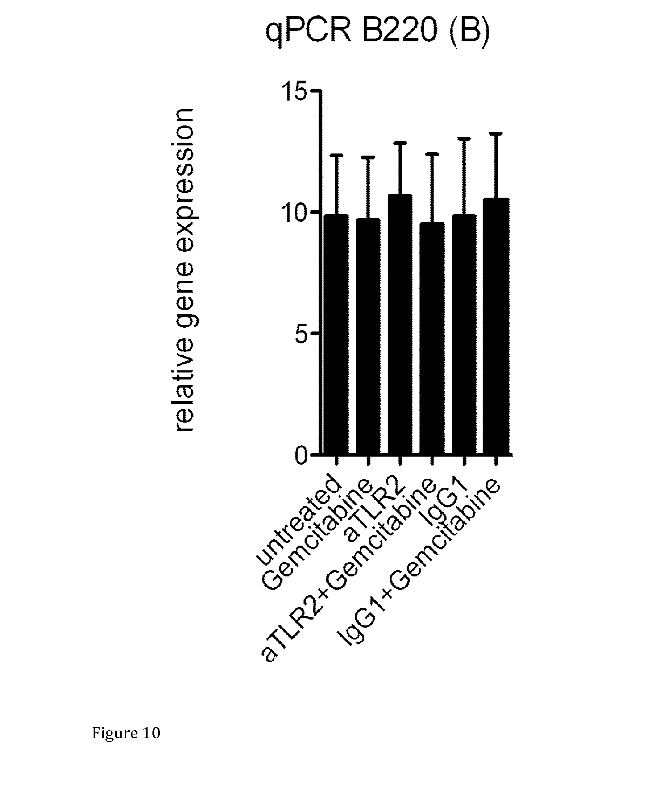

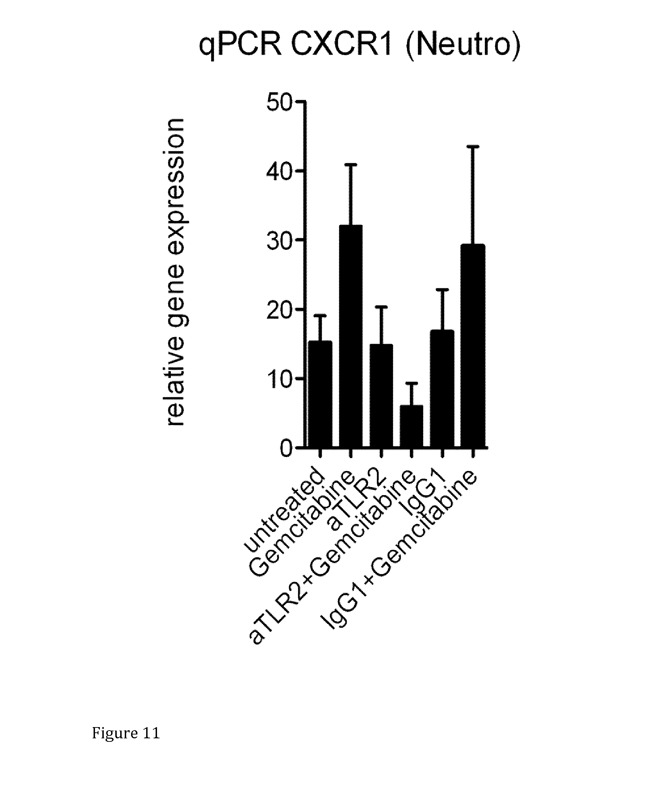

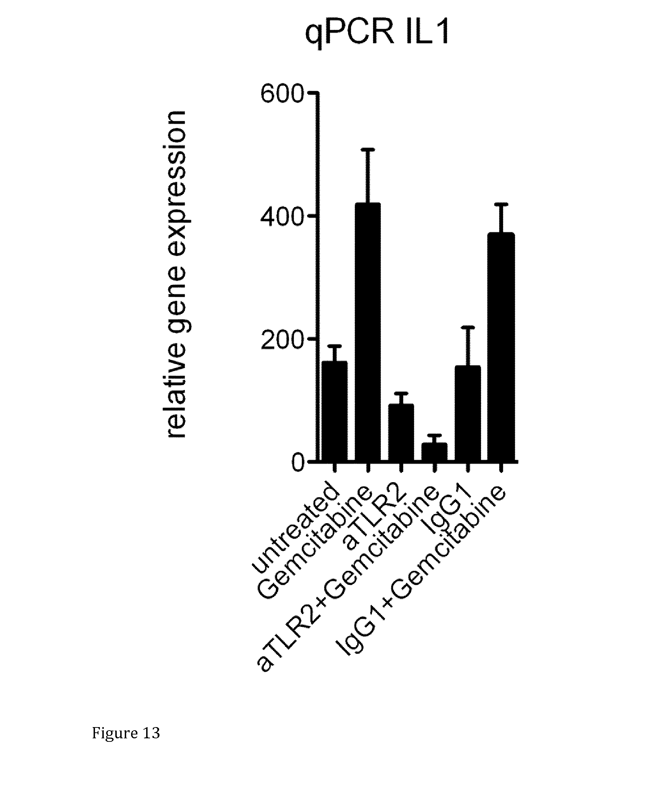

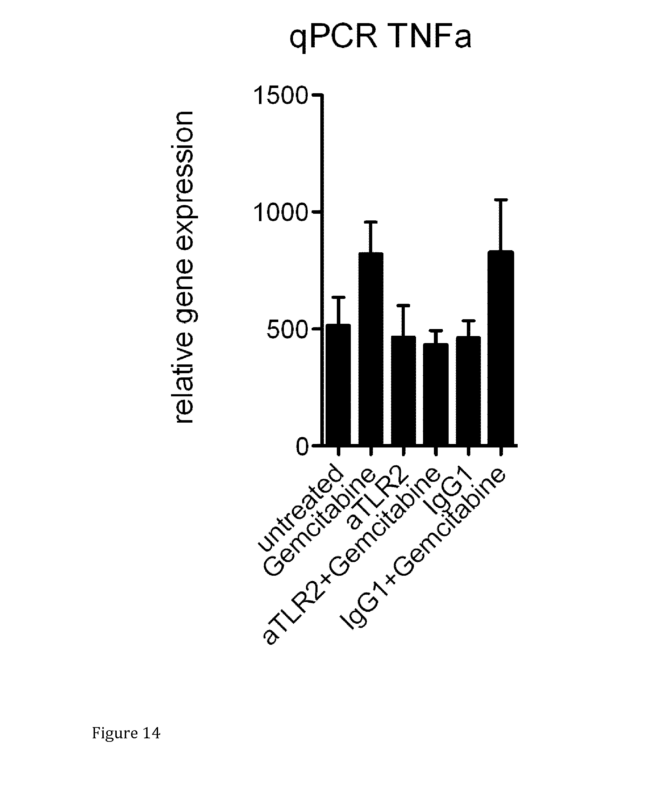

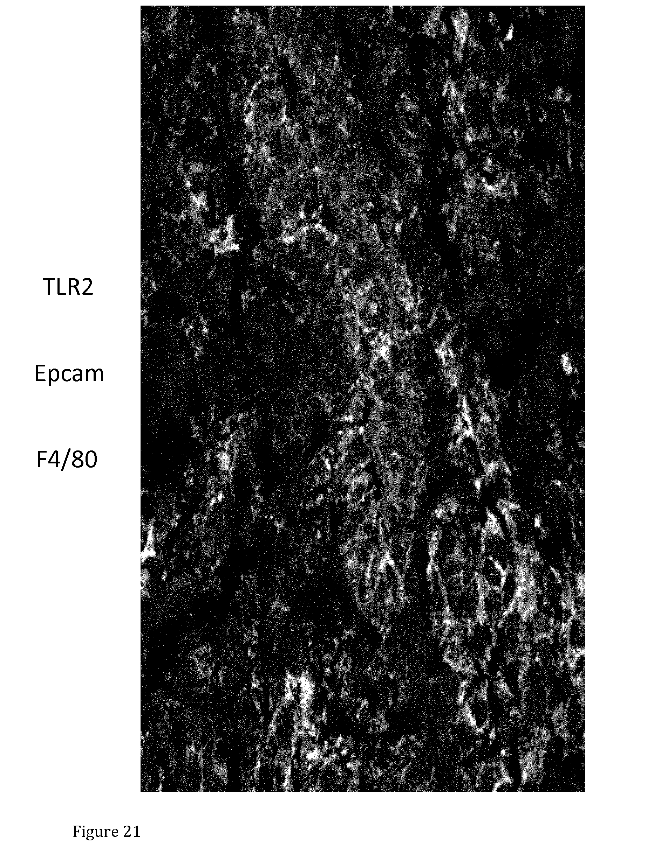

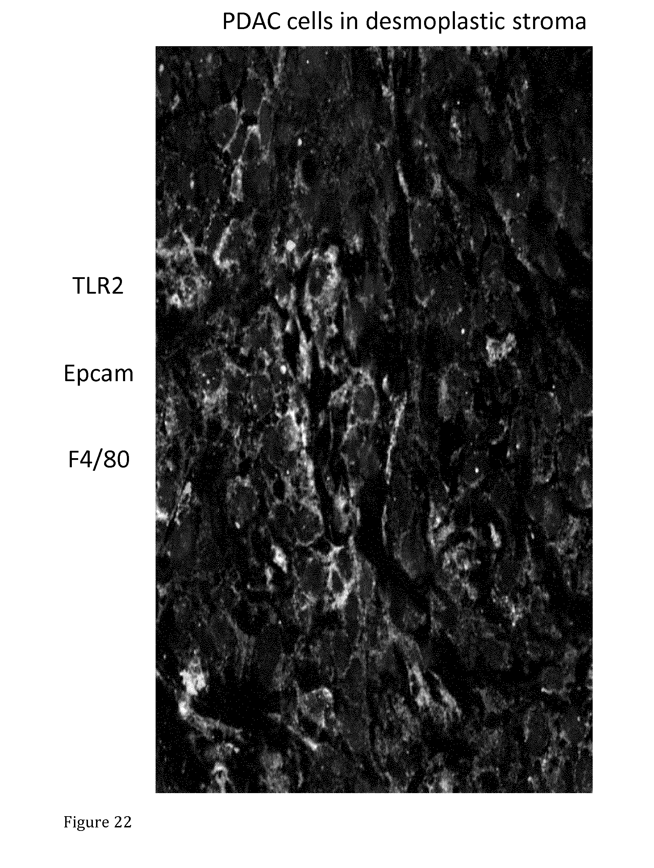

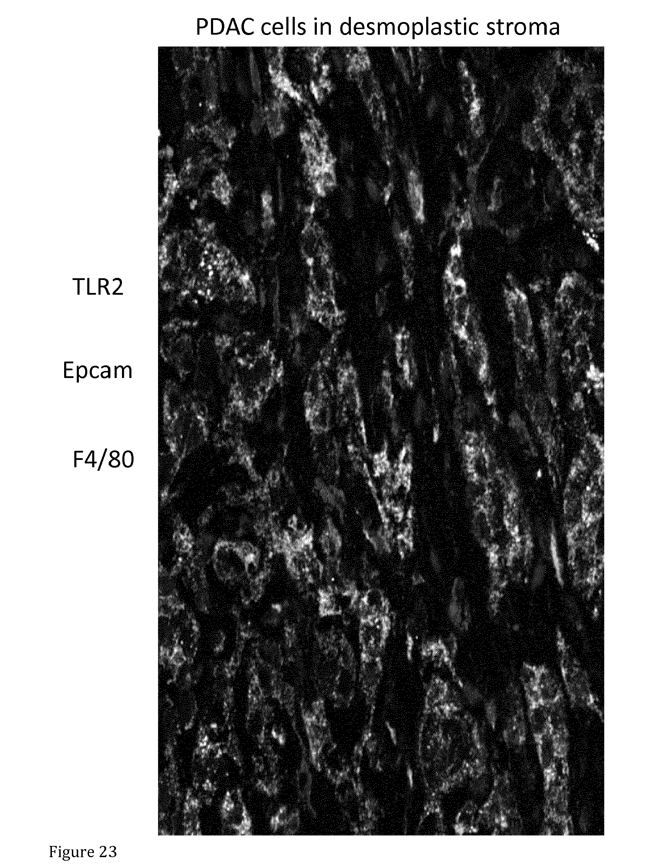

KPC pancreatic tumours are predominantly resistant to gemcitabine. Mice with mean tumour diameters of 5-10mm were enrolled prior to treatment with either saline or gemcitabine (100mg/kg, Q3dx4, i.p.) with and without TLR2 nAb, or TLR2 nAb alone. At the end of treatment, mice were sacrificed and samples collected for analysis. Tumours were analysed by IHC for markers of progression, proliferation, leukocyte infiltrate, angiogenesis and stromal reaction (Figures 1 to 20). To assess the functional vasculature, biotin-conjugated Lycopersicon esculentum lectin was injected i.v prior to euthanasia. Masson's trichrome and α-smooth muscle actin were used to visualize the extracellular matrix and stromal architecture (Figures 21 to 23).

Detailed experimental outline:
Treatment protocol 1 (24 KPC mice):
   1. untreated
   2. Gemcitabine (100mg/kg, every 3^{rd} day for four cycles (Q3dx4), i.p.)
   3. Gemcitabine (100mg/kg, Q3dx4, i.p.) + once weekly TLR2 nAb (1 mg/kg)
   4. OPN301 alone once weekly
   5. Isotype Alone once weekly
   6. Gemcitabine + Isotype

### Treatment protocol 2 (KPC mice):

| | |
|---|---|
| Gemcitabine | 100mg/kg every 3^{rd} day for four cycles (Q3dx4) i.p |
| OPN305 | 10mg/kg weekly i.v. (until death) |
| Abraxane | 10mg/kg Daily for five days, i.v. (Q1dx5) |
| OPN305 F(ab')2 | TBD |

OPN301 + Gem
OPN305 + Gem
OPN305
OPN305 Fab2 + Gem
Untreated
Abraxane
Abraxane + 305

Mice were treated i.v. with 1mg/kg of antibody weekly starting on the first day of gemcitabine therapy. Gemcitabine therapy typically lasts 9 days and the mean survival is 25 days. In order to plan for the amount of antibody to reserve for this study, it was planned to administer OPN301 until day 63 (9 weeks of treatment).

Amount of antibody required (mice assumed to be 20-25g)

| | |
|---|---|
| (6 mice) x (9 treatments) x (25µg/mouse) x (2 groups) | 2700µg |
| Wastage of 25% | 675µg |
| Total | 3375µg |

Tumours were analysed by FACS to quantify leukocyte infiltration using cell surface markers CD8, CD4, NK1.1, CD11b, CD11c, Gr-1, F4/80, CD124 and B220, and markers of T cell and APC activation including T cell: CD69, CD25, CD44, CD62L and CD124; and APC: CD80/86, MHC II, CD206, CD124, CD69, CD200 and Dectin 1. Intracellular cytokine staining for IFNγ, IL-10 and IL-4 was performed on CD4+ and CD8+ TIL and draining lymph node (LN) T cell populations both directly and after magnetic bead separation and ex vivo culture in the presence of PMA and ionomycin for 4 days. Tumour tissue was collected and processed for routine histological analysis and IHC staining for leukocyte subsets. Plasma samples collected from the experiments were screened for cytokine and growth factors (including TNFα, IL-1β, IL-1α, IFNγ, IL-10, IL-4, IL-5, IL-12 (p70), IL-23 (p23), IFNγ, IL-6, CSF-1, TGFβ1, CXCL1, CXCL12 and VEGF) by multiplex assay using the IoC Meso Scale Discovery (MSD) platform. In addition peripheral blood samples were collected and the phenotype and Ly-6C+ and Ly-6Clo circulating monocyte subsets characterised. Plasma samples were measured for circulating levels of OPN301.

The inventors have accumulated and validated a large panel of qPCR primers for analysis of TAM and TDC phenotype (including inducible enzymes: Arginase 1, NOS2, COX2 and IDO; receptors: MR, RANK, CD40, CXCR4, CCR7; cytokines: IL-12p40, IL-12p35, IFNβ, TNFα, IL-1β, IL-1α and IL-10; chemokines: CCL2, CXCL12, CCL17, CXCL1, CXCL10 and CXCL9; and signalling proteins: PAI2, SOCS1/3, IkBa, A20 and BCL3). T cells will also be characterised by Gata-3, ROR-γT, T-BET and FoxP3. Tumour associated macrophages are recruited into tumours as monocytes from the bloodstream by chemotactic cytokines and growth factors such as CCL2 (MCP-1), M-CSF (CSF-1), vascular endothelial growth factor (VEGF), angiopoietin-2 and CXCL12 (SDF1). TAM acquire a specific phenotype that is oriented toward tumour growth, angiogenesis and immune-suppression and many studies have shown a positive correlation between the number of TAM and poor prognosis in human tumours. There is also increasing evidence that TAM contribute to suppression of anti-tumour immune responses, in particular the M2-phenotype of TAM is associated with increased expression of arginase 1 and indoleamine 2,3-dioxygenase (IDO) that inhibit T-cell proliferation, as well as immunosuppressive cytokines IL-10 and TGFβ. Blockade of TAM recruitment, for example by the genetic deletion of CSF-1, blocks tumor growth, angiogenesis, and metastasis in experimental models of cancer. Tolerant DCs (tDCs) block DC activation. The increased ratio of tolerant DCs/activated DCs promotes formation of regulatory T-cells (Tregs) and inhibits effector T-cells. Leukocytes commonly infiltrate solid tumours, and have been implicated in the mechanism of spontaneous regression in some cancers.

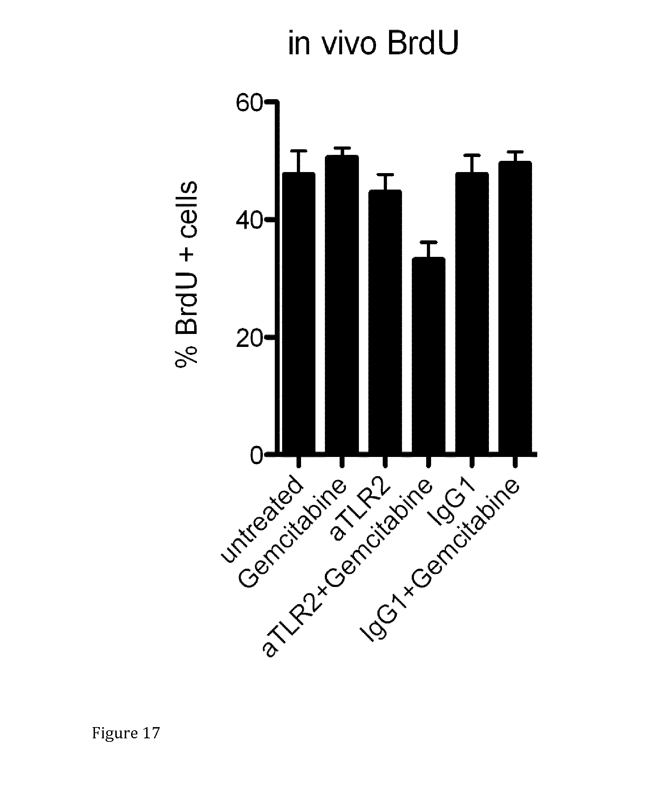

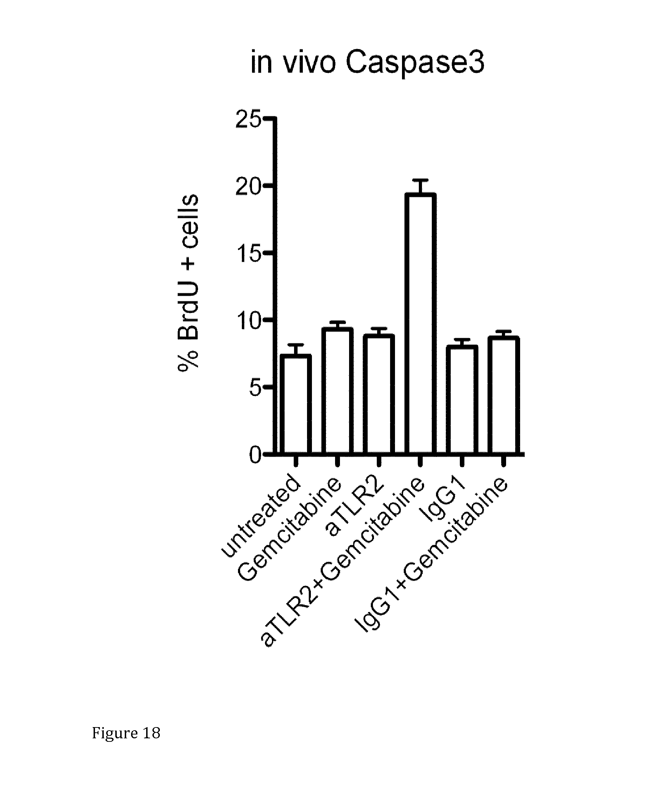

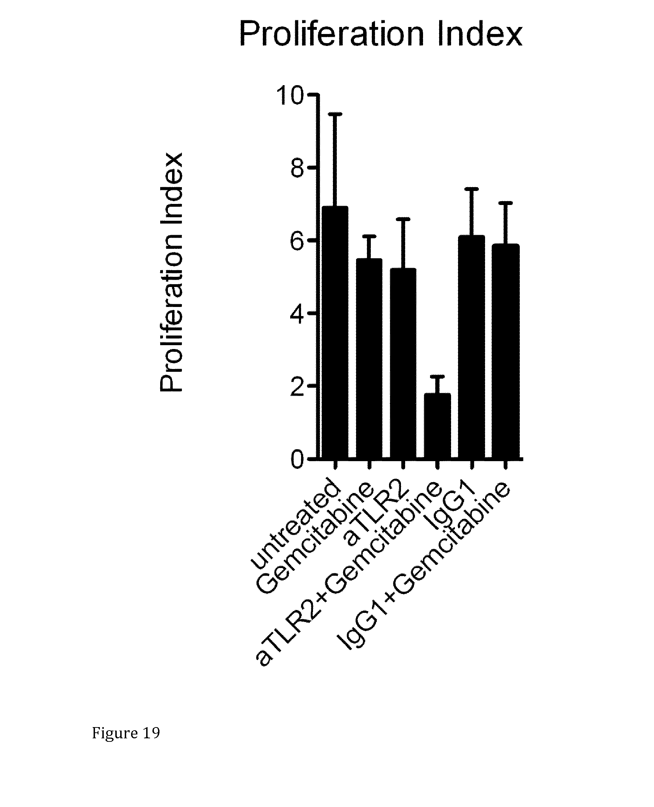

Tumour apoptosis and proliferation was assessed by injection of the thymidine analog 5-bromo-2'-deoxyuridine (BrdU) (Figure 17) followed by Caspase 3 staining (Figure 18) to assess the proliferation index (Figure 19). 250 µl of BrdU at 50 µg/g of total body weight was injected ip. 90 minutes after BrdU injection, mice were sacrificed and each pancreas was processed and stained. Histological analysis of pancreases was carried out by standard procedures. Specimens were harvested from time-matched animals and fixed in 4 % v/v buffered formalin overnight at 4°C. The following day, the organs were progressively dehydrated in gradient alcohols (30 minutes at room temperature in 30, 50 and 70 % v/v ethanol). Tissues were embedded in paraffin and sections were cut with a microtome (5 µm thickness) and prepared by the Barts Cancer Institute pathology department. The BrdU antibody from the BrdU labelling and detection kit was used (Roche - 11299964001) and the peroxidase activity was visualised using SIGMAFastTM 3,3'-diaminobenzidine (DAB) with metal enhancer at a final concentration of 0.5 mg/ml DAB, 0.2 mg/ml cobalt chloride, 0.3 mg/ml urea hydrogen peroxide, 0.05 M Tris buffer and 0.15 M sodium chloride (Sigma-Aldrich - D0426).

Sections were stained using a standard streptavidin-peroxidase complex technique and the following steps were carried out at room temperature using immunoboxes for washes. Sections were deparaffinised and rehydrated. To improve the exposure of antigen sites on the sections, an antigen retrieval step is usually necessary, which is specific for each primary antibody used. The heat-mediated method was used, which involves immersing the sections in antigen citric acid based unmasking solution (Vector Labs - H-3300) and heating for 9 minutes in a microwave. Sections were allowed to cool down at room temperature for at least 20 minutes and washed three times for 3 minutes each wash in PBS. Sections were circled with ImmEdgeTM hydrophobic barrier pen (Vector Labs - H4000) and blocked for nonspecific binding with PBS/BSA/Goat serum for 1 hour at room temperature in a humidified chamber. Sections were incubated in primary antibody or isotype control diluted in blocking solution overnight at 4°C in a humidified chamber. The following day, the sections were again washed three times for 3 minutes each wash in PBS and subsequently the biotinylated secondary antibody was applied and the sections were incubated for 45 minutes at room temperature in a humidified chamber. Sections were again washed three times for 3 minutes each wash in PBS and the endogenous peroxidase activity was quenched by incubating the sections in 0.3 % v/v H₂O₂ diluted in 100 % v/v methanol for 20 minutes at room temperature. The sections were then washed three times for 3 minutes each wash in PBS and avidin biotinylated peroxidase complex (Vector Labs - Vectastain Elite ABC Kit - PK-6100) was added onto the sections and incubated for 30 minutes at room temperature in a humidified chamber. The sections were once again washed three times for 3 minutes each wash in PBS and the peroxidase activity was visualised by using SIGMAFastTM 3,3'-diaminobenzidine (DAB) at a final concentration of 0.7 mg/ml DAB, 0.67 mg/ml urea hydrogen peroxide and 0.06 M Tris buffer (Sigma-Aldrich - D4418). DAB solution was applied on each section for at least 3 minutes or until a brown colour developed. Subsequently, sections were briefly washed in water to stop DAB development and counterstained by dipping in haematoxylin solution (Sigma-Aldrich - GSH316) for 30 seconds, washed in water and dipped 10 times in ammonium hydroxide (44.4 mM in water) for acid differentiation. Sections were dehydrated by dipping them 20 times in isobutanol (Fisher Scientific - B/5100/PB17) and incubated again in fresh isobutanol for 4 minutes and then twice in xylene for 5 minutes. Slides were air-dried and mounted with DPX mounting medium (Fisher Scientific - D/5319/05). Cleaved Caspase 3 - cell signalling, Cat No 9664. rabbit IgG, 1:200; Isotype control rabbit IgG, BD Bioscience 550875, 1:200.

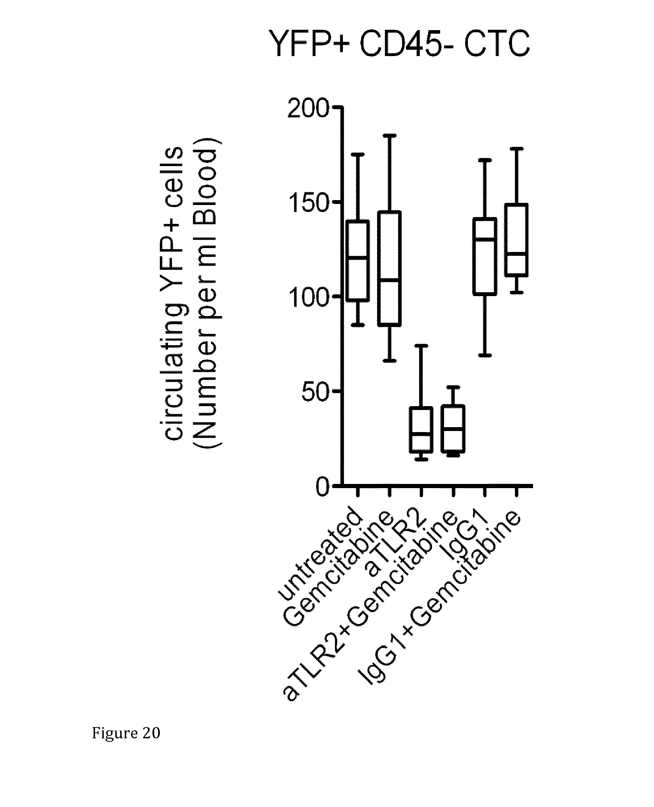

Enriched blood samples were stained for various markers prior to flow cytometric analysis or flow sorting. For analysis and sorting based on endogenous YFP and surface markers, primary antibodies (1:100) and secondary antibodies (1:50) were incubated with cells in 10%FCS /DMEM/F12 for 20 minutes at 4°C (Figure 20). Primary pancreas samples and PanIn and PDAC cell lines from the reporter mice were used as positive controls. Isotype controls were also run.

### Results

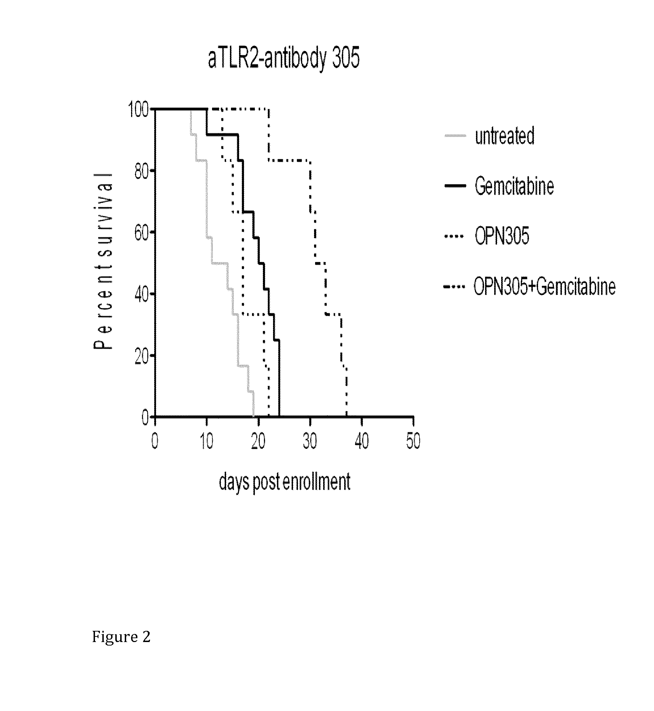

As shown in Figures 1 and 2, the percentage survival of mice increased following treatment of mice having mean tumour diameters of 5-10mm with TLR2 neutralising antibody (nAb) OPN301 (Figure 1) or OPN-305 (Figure 2). This effect was synergistically enhanced when the TLR2 nAb was combined with gemcitabine.

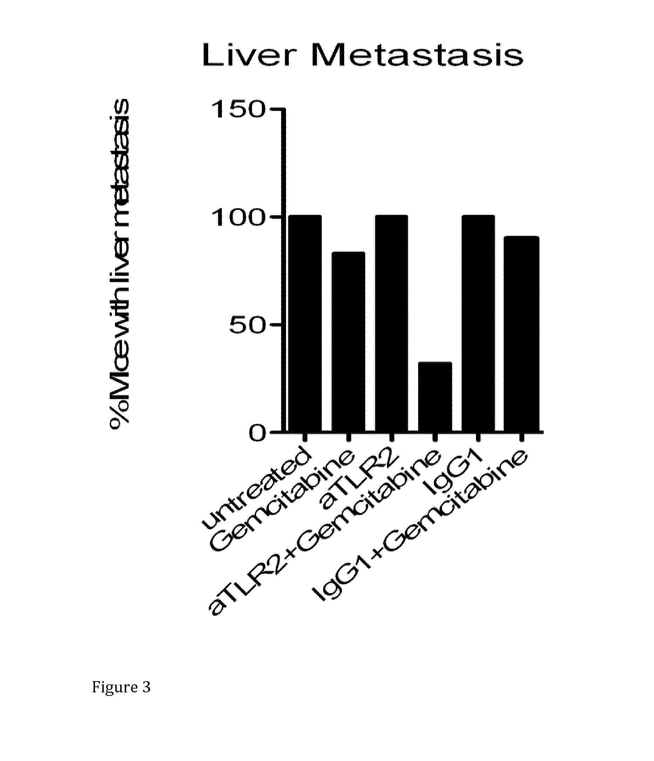

The percentage of mice with liver metastasis was significantly reduced following treatment of mice with gemcitabine and TLR2 nAb (Figure 3).

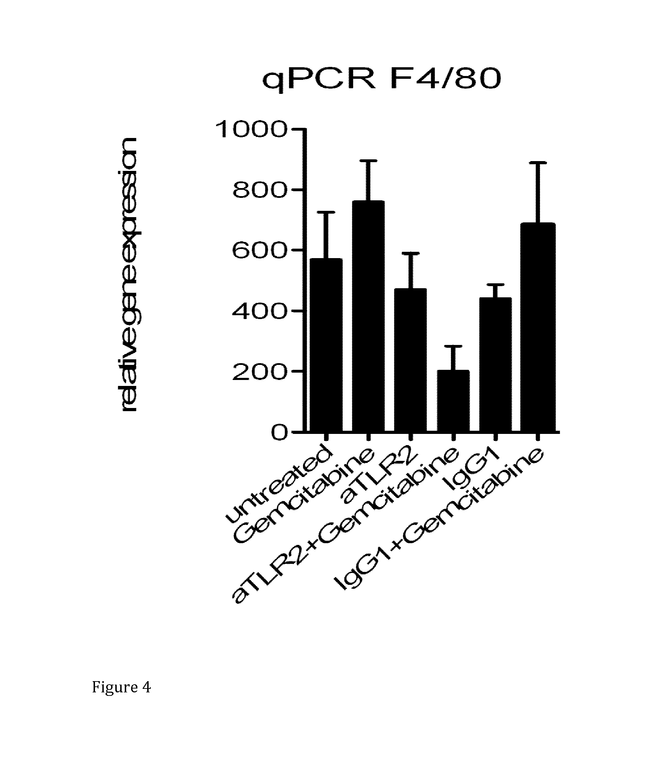

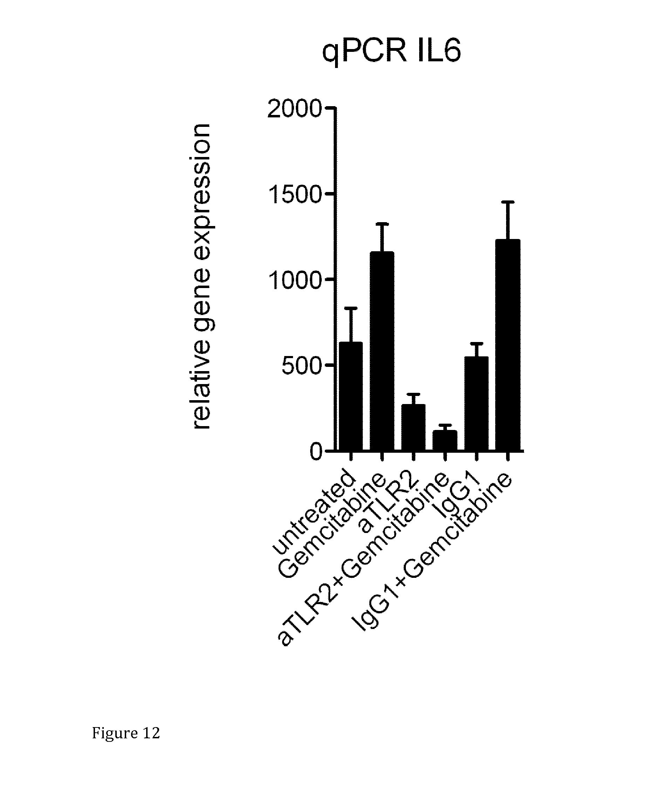

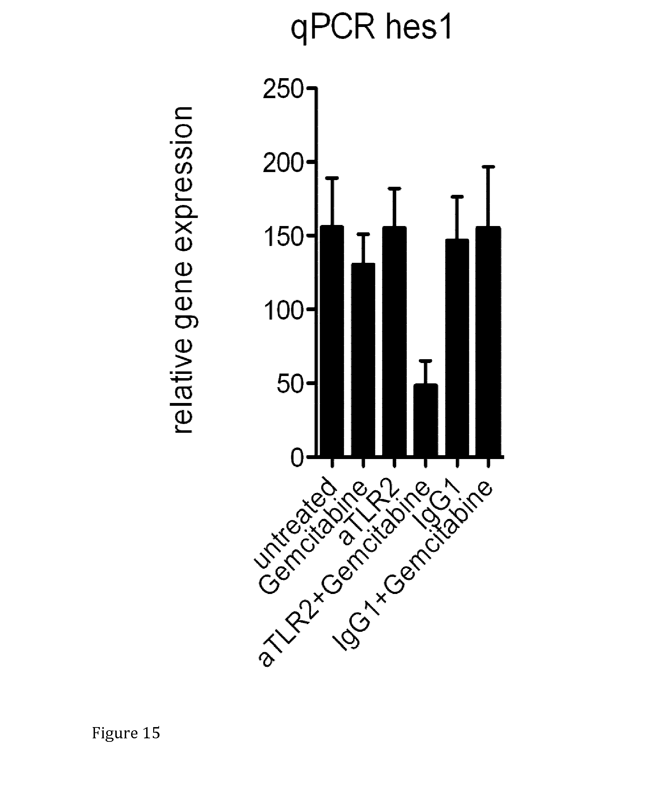

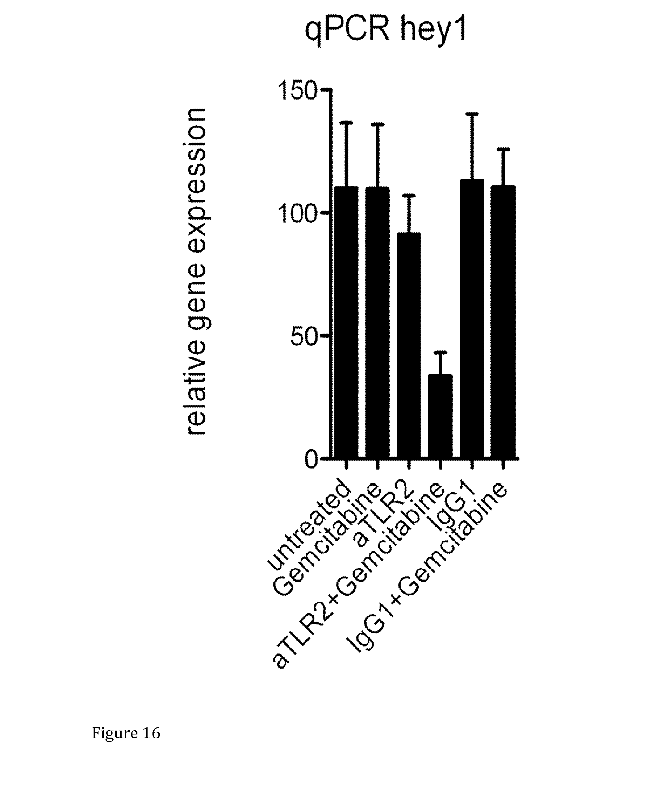

The results of analysis of tumours by FACS to quantify leukocyte infiltration using cell surface markers and markers of T cell and APC activation are shown in Figures 4 to 11. The results for cytokines and growth factors are shown in Figures 12 to 14 and the results for transcription factors hes1 and hey1 are shown in Figures 15 and 16. qPCR for F4/80, CD8a, CCR1, CCR3, NK1.1, CD208, B220 and CXCR1 is used as an initial crude read-out to determine differences on total PDAC RNA for differential recruitment of leukocytes. Inflammatory cytokines have been described as tumour drivers in the KPC mouse model. Assessment of inflammatory cytokines is a possibility of assessing infiltrating leukocytes and their production of these cytokines. Hes1 andhey1 are markers for PDAC progression and are used to define the impact of treatment on disease progression.

As shown in Figures 17 to 19, there was an increase in tumour apoptosis and a decrease in proliferation in mice treated with TLR2 nAb. This effect was significantly enhanced when the TLR2 nAb was combined with gemcitabine when compared with mice treated with either TLR2 nAb or gemcitabine alone. Apoptosis was assessed by cleaved caspase 3 and proliferation was assessed by Ki67 expression. The calculated proliferation index provides an indication of the tumour turnover. The use of TLR2 antibodies reduced tumour cell proliferation and increased caspase 3. The net effect is reduced proliferation and therefore a delay in cancer progression. As tumour cells express TLR2 in this model, the effect might be the result of the TLR2 antagonist increasing sensitivity to gemcitabine, or in addition to the chemotherapeutic effect the TLR2 antagonist may provide an additional immunostimulatory effect which increases the overall immune response.

Figure 21 shows IF using a TLR2 antibody from Santa Cruz. PDAC lesions are expressing TLR2 as demonstrated in the co-localisation with Epcam. No TLR2 expression was noticed on F4/80 positive infiltrating macrophages. Staining demonstrated a lack of staining for TLR on myeloid cells, but with significant co-localization on the EpCam positive malignant cells.

All documents referred to in this specification are herein incorporated by reference. Modifications and variations to the described embodiments of the inventions will be apparent to those skilled in the art, without departing from the scope of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the art are intended to be covered by the present invention.

## Claims

1. A composition comprising a TLR2 antagonist for use in the treatment or prophylaxis of pancreatic cancer in a subject.

2. The composition as claimed in claim 1 wherein the TLR2 antagonist specifically binds to an epitope comprising leucine rich repeat regions 11 to 14 of TLR2.

3. The composition as claimed in claim 1 or 2 wherein the TLR2 antagonist specifically binds to a non-continuous epitope comprising amino acid residues His318, Pro320, Arg321, Tyr323, Lys347, Phe349, Leu371, Glu375, Tyr376 and His398 of SEQ ID NO: 1.

4. The composition as claimed in any one of claims 1 to 3 wherein the TLR2 antagonist specifically binds to a non-continuous epitope comprising amino acid residues His318, Pro320, Gln321, Tyr323, Lys347, Phe349, Leu371, Glu375, Tyr376 and His398 of SEQ ID NO: 2.

5. The composition as claimed in any one of claims 1 to 4 wherein the TLR2 antagonist is selected from the group consisting of an antibody or an antigen binding fragment thereof, a protein, a peptide, a peptidomimetic, a nucleic acid, a carbohydrate, a lipid and a small molecule.

6. The composition as claimed in claim 5 wherein the TLR2 antagonist is an antibody or an antigen binding fragment thereof.

7. The composition as claimed in claim 6 wherein the antibody or antigen binding fragment comprises a heavy chain variable region comprising a complementarity determining region (CDR) 1 region comprising the amino acid sequence of SEQ ID NO:3, a CDR2 region comprising the amino acid sequence of SEQ ID NO:4 and a CDR3 region comprising the amino acid sequence of SEQ ID NO:5, and a light chain variable region comprising a CDR1 region comprising the amino acid sequence of SEQ ID NO:6, a CDR2 region comprising the amino acid sequence Gly-Ala-Ser and a CDR3 region comprising the amino acid sequence of SEQ ID NO:7.

8. The composition as claimed in claim 7 wherein the antibody or antigen binding fragment comprises a heavy chain variable region comprising the amino acid sequence of SEQ ID NO:8, or a sequence which has at least 90% amino acid sequence identity to the amino acid sequence of SEQ ID NO:8, and a light chain variable region comprising the amino acid sequence of SEQ ID NO:9, or a sequence which has at least 90% amino acid sequence identity to the amino acid sequence of SEQ ID NO:9.

9. The composition as claimed in claim 6 or 7 wherein the antibody or antigen binding fragment comprises a light chain variable domain comprising an amino acid sequence of SEQ ID NO:10, or a sequence which has at least 90% amino acid sequence identity over a length of at least 20 amino acids of the amino acid sequence of SEQ ID NO:10, and a heavy chain variable domain comprising an amino acid sequence of SEQ ID NO:11, or a sequence which has at least 90% amino acid sequence identity over a length of at least 20 amino acids of the amino acid sequence of SEQ ID NO:11, wherein the antibody or antigen binding fragment specifically binds to TLR2 and does not bind to CD32.

10. The composition as claimed in claim 9 wherein the antibody or antigen binding fragment comprises a heavy chain variable region comprising the amino acid sequence of SEQ ID NO:13, or a sequence which has at least 90% amino acid sequence identity to the amino acid sequence of SEQ ID NO:13, and a light chain comprising the amino acid sequence of SEQ ID NO:12, or a sequence which has at least 90% amino acid sequence identity to the amino acid sequence of SEQ ID NO:12.

11. The composition as claimed in any one of claims 1 to 10 wherein the treatment comprises the simultaneous, separate or sequential administration of a chemotherapeutic agent selected from the group consisting of gemcitabine, cyclophosphamide, abraxane, fluorouracil (5FU), FolFox, Folfiri and Folfirinox.

12. The composition as claimed in claim 11 wherein the chemotherapeutic agent is gemcitabine.

13. The composition as claimed in any one of claims 1 to 12 wherein the pancreatic cancer is metastatic.

14. A pharmaceutical composition comprising a TLR2 antagonistic antibody or an antigen binding fragment thereof, a pharmaceutically acceptable carrier and gemcitabine, wherein the TLR2 antagonistic antibody or antigen binding fragment comprises a heavy chain variable region comprising a complementarity determining region (CDR) 1 region comprising the amino acid sequence of SEQ ID NO:3, a CDR2 region comprising the amino acid sequence of SEQ ID NO:4 and a CDR3 region comprising the amino acid sequence of SEQ ID NO:5, and a light chain variable region comprising a CDR1 region comprising the amino acid sequence of SEQ ID NO:6, a CDR2 region comprising the amino acid sequence Gly-Ala-Ser and a CDR3 region comprising the amino acid sequence of SEQ ID NO:7.

15. A screening method for the identification of compounds for use in treatment or prevention of pancreatic cancer, the method comprising the steps of:
(a) providing candidate compounds having binding specificity for TLR2;
(b) contacting the candidate compounds with TLR2; and
(c) identifying compounds which bind to TLR2 within the region of amino acid residues His318, Pro320, Arg321 or Gln321, Tyr323, Lys347, Phe349, Leu371, Glu375, Tyr376 and His398 of SEQ ID NO: 1 or SEQ ID NO:2;
wherein binding in this region is indicative of utility of the compound in the treatment or prevention of pancreatic cancer.
